# EUROPEAN PATENT SPECIFICATION

(11) **EP 0 531 537 B1**
(45) Date of publication and mention of the grant of the patent: **11.09.1996**
(21) Application number: 92905744.6
(22) Date of filing: 04.03.1992
(51) Int. Cl.: C07K 7/08, C07K 1/02, A61K 38/07, A61K 38/55

(54) **HIRUDIN ANALOGUE OR SALT THEREOF, PRODUCTION THEREOF, AND ANTICOAGULANT CONTAINING THE SAME AS ACTIVE INGREDIENT**
HIRUDINANALOG ODER DESSEN SALZ, DESSEN HERSTELLUNG UND DAS ALS AKTIVEN BESTANDTEIL ENTHALTENDE ANTIKOAGULANZ
ANALOGUE D'HIRUDINE OU SEL DE CE COMPOSE, SA PRODUCTION, ET ANTICOAGULANT LE CONTENANT EN TANT QU'INGREDIENT ACTIF

(30) Priority: 05.03.1991 JP 63909/91
(43) Date of publication of application: 17.03.1993
(73) Proprietor: JAPAN ENERGY CORPORATION, Minato-ku, Tokyo (JP); FUJI YAKUHIN KOGYO KABUSHIKI KAISHA, Takaoka-shi Toyama 933 (JP)
(72) Inventor: MURAMATSU, Ryo, Nippon Mining Co., Ltd., Saitsma 335 (JP); SUKESADA, Akiko, Nippon Mining Co., Ltd., Saitama 335 (JP); MISAWA, Satoru, Nippon Mining Co., Ltd., Saitama 335 (JP); NIKUI, Eriko, Fuji Yakuhin Kogyo K K Kenyusho, Toyama 933 (JP); WADA, Koichi, Fuji Yakuhin Kyogo K K Kenyusho, Toyama 933 (JP); NAKANO, Masaharu, Fuji Yakuhin Kog. K K Kenkyusho, Toyama 933 (JP); MORIKAWA, T., Fuji Yakuhin Kogyo KK Kenkyusho, Toyama 933 (JP); KOBASHI, Kyoichi, 22, Hasumachi, Toyama 931 (JP)
(74) Representative: Davies, Jonathan Mark
(86) International application number: JP9200253
(87) International publication number: WO9215610

(56) References cited:
- WO-A-90/06128
- JP-A-63 215 698
- JOURNAL OF BIOLOGICAL CHEMISTRY. (MICROFILMS) vol. 265, no. 16, 5 June 1990, BALTIMORE, MD US pages 9314 - 9318 C. NIEHRS ET AL. 'Conversion of Recombinant
- Hirudin to the Natural Form by in Vitro Tyrosine Sulfatation'
- Thromb. Haemostasis, Vol. 63, No. 2 (1990), JOHN L. KRSTENANSKY, et al. "Development of MDL 28, 050, a small stable antithrombin agent based on a functional domain of the leech protein, hirudin" pp. 208-214.
- Thromb. Res., Vol. 52, No. 2 (1988), JOHN L. KRSTENANSKY, et al. "Comparison of hirudin and hirudin PA C-terminal fragments and related analogs as antithrombin agents" pp. 137-141.

## Description

### Technical Field

The present invention relates to hirudin variants or their salts, method for their production and anti-coagulants having said compounds as active ingredients. The hirudin variants or their salts in the present invention are useful as drugs for pharmacological therapy of acute deep venous thrombosis, pulmonary thromboembolism, acute arterial embolism of limbs, myocardial infarction and intravascular coagulation on infection.

### Background Art

Natural hirudin is a mixture of peptides composed of 65 or 66 amino acids and is secreted from salivary glands of medicinal leech in very small amount. A variant called HV-1 is the first hirudin isolated from leech. A variant called HV-2 differs from aforesaid HV-1 in 9 amino acids, and HV-3 is identical with HV-2 up to 32nd serine and differs in 10 amino acids including an additional 63rd alanine on C-terminal.

Further, existence of tyrosine of which phenolic hydroxide residue is sulfated as shown in the following formula 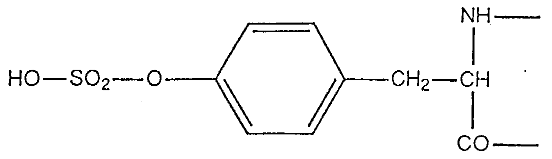 or

-[Tyr(SO₃H)]-

has been confirmed.

It has been reported that the anti-thrombin activity increases about 10 times by existence of sulfate on the tyrosine residue.

So far, it is very difficult to manufacture polypeptides having sulfated tyrosine in the molecule. When chemical introduction of sulfate group on tyrosine residue in polypeptide obtained by methods such as recombinant DNA technology is considered, in which case the amino acid sequence is long, it is very difficult to sulfate targeted tyrosine selectively without giving any influence on other amino acids. It also requires drastic reaction conditions which often cause disruption of peptide bonds and it is difficult to obtain the sulfated compound in satisfactory yield. For this reason, hirudins under development at present as anti-coagulants are non-sulfated ones and have low activities.

Although the therapeutic application of hirudin as an anti-coagulant is thought to be effective, being a foreign compound, allergic symptom such as shock and eczema are well possible. However, it is thought to be possible to reduce allergic response by decreasing the administration dose or shortening the amino acid sequence of polypeptide. The aim of the present invention is to provide hirudin variants with higher anti-thrombin activity by sulfating the hydroxyl group of tyrosine residue in the molecule.

### Disclosure of Invention

The present invention relates to hirudin variants or their salts having following amino acid sequence in formula (I) as a part of their sequence or as all of their sequence. Phe - Glu - A - Ile - Pro - B - Tyr(R) - Tyr(R)     (I) [In the formula, A represents Glu or Pro, B represents Glu, Tyr(R), Glu-Asp or Glu-Tyr(R), and (R) represents the hydroxy group or its sulfated ester (-O-SO₃H) of tyrosine residue.]

Further, the present invention relates to the method of manufacturing of the hirudin variants or their salts characterized by sulfating the hydroxyl group of tyrosine residue in the amino acid sequence of hirudin variants or their salts having the following formula (II) as a part or as all of the amino acid sequence. Phe - Glu - A - Ile - Pro - B - Tyr - Tyr (II) [In the formula, A represents Glu or Pro, B represents Glu, Tyr, Glu-Asp or Glu-Tyr, respectively.]

The sulfation in the present invention may be carried out by reacting hirudin variants or their salts with aryl sulfotransferase in the presence of sulfate group donors, with sulfur trioxide complex, or with sulfuric acid and dicyclohexylcarbodiimide.

One of the important points of the present invention is that the recognition activity against tyrosine by aryl sulfotransferase is improved by substituting 1 or 2 amino acids adjacent to the tyrosine residue in C terminal of natural hirudin to tyrosine. With the polypeptides used in the present invention, the present inventors have confirmed that the sulfation reaction by aryl sulfotransferase will hardly take effect without the aforesaid treatment.

Another important point of the present invention is, if a peptide contains a sequence of 8 amino acids starting from 56th amino acid from N-terminal to C-terminal of natural hirudin, or if it is composed of solely aforesaid sequence of 8 amino acids, or if the both ends of it are substituted by protecting group such as succinyl group or amino group, it should show a high anti-thrombin activity as long as it is sulfated. Particularly, a compound composed of only 8 amino acids according to formula (I), having N-terminal protected by succinyl group and two tyrosine residues sulfated both hydroxyl group thereof, is confirmed to have an extremely high anti-thrombin activity.

The hirudin variants in the present invention are the peptides having aforesaid amino acid sequence and having high anti-thrombin activity.

The C-terminal of the compounds in the present invention are abovementioned -Tyr(R), -Tyr(R)-Leu or -Tyr(R)-Asp. Further, aforesaid -Tyr(R), -Tyr(R)-Leu or -Tyr(R)-Asp may have the following substituents.
Amide, lower alkyl(C₁ - C₅) amide, [e. g. -NHCH₃, -NHC₂H₅], amino aoids [e.g. natural amino acids, D-Glu, α-amino adipic acid, α-amino suberic acid(Asu)], lower alkyl(C₁ - C₅) ester of amino acids [e.g. Glu-OC₂H₅, Glu(OC₂H₅)OC₂H₅, Asu (OMe)-OMe], amino acid amide [e.g. Glu-NH₂, -Gln-NH₂, Asu(NH₂)NH₂], lower alkyl(C₁ - C₅) amide of amino acids [e.g. Glu-NHC₂H₅, -Gln-NHC₂H₅], amino sulfonic acids [e.g. -NH-CH₂-SO₃H, taulin (Tau), -Nli-(CH₂)₃-SO₃H], aminosulfone amide [e.g. -NH-CH₂-SO₂NH₂, Tau-NH₂], amino alcohol [e.g. -NH(CH₂)₂-OH, -NH(CH₂)₃-OH, Leu-o1], amino phosphoric acid [e.g. -NHPO (OH)₂] amino phosphoric acid ester [e.g. -NHPO (OC₂H₅)₂, -NHPO (OPh)₂] or amino phosphone amide [e.g. -NHPO (NH₂)₂].

And, the following acyl group may be used as protecting groups of N-terminal amino group.
Alkanoyl [e.g. acetyl (CH₃CO-), butyryl (CH₃CH₂CH₂CO-), isobutyryl ((CH₃)₂CHCO-)], substituted alkanoyl [e.g.lactinyl(CH₃CH(OH)CO-)], carboxy alkanoyl [e.g. succinyl (HOOCCH₂CH₂CO-), glutaryl (HOOC(CH₂)₃CO-)], substituted carboxy alkanoyl [e.g. malicyl (HOOCCH(OH)CH₂CO-)], alkoxycarbonyl alkanoyl [e.g. ethoxy carbonyl propionyl (EtOOCCH₂CH₂CO-)], carbamoyl alkanoyl [e.g.carbamoyl propionyl (H₂NOCCH₂CH₂CO-)], alkenoyl [e.g. acryl (CH₂=CHCO-), oleonyl (CH₃(CH₂)₇CH=CH(CH₂)₇CO-)], carboxy alkenoyl [e.g.3-carboxy-cis-propenoyl, 3-carboxy-trans-propenoyl (HOOCCH=CHCO-)], alkoxycarbonyl alkenoyl [e.g.ethoxy carbonyl acryloyl EtOOCCH=CHCO-)], or carbamoylalkenoyl [e.g. carbamoylacryloyl (H₂NOCCH=CHCO-)]

The hirudin variants of the present invention may form salts with acids or bases etc.

Salts in the present invention may be the followings: hydrochloride,sulfate, p-toluene sulfonate, phosphate, formic acid salt, malonic acid salt, succinic acid salt, lactic acid salt, oxalic acid salt, tartaric acid salt, acetic acid salt, trifluoroacetic acid salt, sodium salt, potassium salt, magnesium salt, barium salt, calcium salt, ammonium salt, piperidine salt, morpholine salt, dimethyl amine salt, diethyl amine salt,etc.

The polypeptides including the amino acid sequence shown in aforementioned formula (II) can easily be produced by various known methods. Such methods includes chemical synthesis methods like solid phase method and liquid phase method, recombinant DNA method and the combination of these method.

The method of sulfation using aryl sulfotransferase in the present invention has advantage that it can sulfate tyrosine residues specifically in polypeptides of long amino acid sequence in mild condition so that it does not affect other amino acids. There is no particular limitation to the enzyme used for the sulfation as long as it has aryl sulfotransferase activity, for instance, the one derived from human enterobacterium (Eubacterium A-44).

Preferable examples of sulfate group donors are aryl sulfates or their salts, for example, phenyl sulfate, p- or m- nitro phenyl sulfate, p- or m- acetyl phenyl sulfate, tyramine sulfate, p-nitro catechol sulfate, p-nitro catechol disulfate, pico sulfate, phenolphthalein disulfate, 4-methyl unberiferril sulfate; 1- or 2-naphthyl sulfate, 4-nitro-1-naphthyl sulfate, 4-fenantoryl sulfate, or their alkali metal salts.

Reaction temperature and pH should be optimized depending on the nature of polypeptide and aryl sulfotransferase, however, the present inventers have found preferable conditions at the temperature of 25 - 37 °C and at the pH of 8 - 9.

When the reaction is over, the sulfated compound may easily be separated and collected from unreacted materials by high performance liquid chromatography method under appropriate condition. Recovered unreacted materials may be re-used for sulfation of polypeptide by feeding them back to the reaction system.

On the other hand, those polypeptide chains of relatively short amino acids including the amino acid sequence shown in aforementioned formula (I) may be generally chemically synthesized by solid phase or liquid phase method. Hydroxyl group of tyrosine residue of these peptides may be sulfated by chemical method using sulfating reagents. The sulfation may be carried out by treating aforementioned polypeptide chain at around room temperature, in the presence of solvents, such as pyridine or dimethyl formamide, with 10 - 500 equivalent amount excess of sulfur trioxide complex, such as pyridine-sulfur trioxide, dioxane-sulfur trioxide, trimethylamine-sulfur dioxide, triethylamine-sulfur trioxide, dimethylaniline-sulfur trioxide, thioxane-sulfur trioxide, bis (2-chloroethyl)ether-sulfur trioxide, 2-methylpiridine-sulfur-trioxide, quinoline-sulfur trioxide, dimethylformamide-sulfur trioxide. 'As an alternative method, sulfation may be done by condensation method by treating excess of sulfuric acid and dicyclohexylcarbodiimide with aforementioned polypeptide chain at around room temperature.

When compounds obtained in the present invention are going to be used as pharmaceuticals, for example, they may be administered orally, or subcutaneously, intravenously, intramuscularly, intraarterialy by injection, or non-orally like through mucous. The dose of 0.1 - 1000mg for an adult per a day is suitable. The total amount may be administered in 1 to several times. However, naturally, the amount may be properly increased or decreased upon necessity. For oral administration, they may be used in form of tablet, capsule or granule, which are prepared by using pharmaceutically acceptable additives, diluent, carrier, excipients etc. For non-oral administration, the compounds may be prepared into injectable formulation of solution or suspension, using sterilized solution of water or oils, detergents, other' pharmaceutically acceptable additives, pharmacologically acceptable diluents or carriers, etc. Similarly, using pharmaceutically acceptable additives, diluents, carriers or excipients etc., the compounds maybe prepared into suppository or into formulation that enables absorption through skin or mucous.

### Brief Description of the Drawings

- Fig. 1: shows relation of recovery of amino acid synthesized in example 1 and its phenylthiohydantoin(PTH) derivatives.
- Fig. 2: shows profile of HPLC analysis chromatogram of each peptide synthesized in example 1.
- Fig. 3: shows profile of HPLC analysis chromatogram of sulfated compound described in example 2.
- Fig. 4: shows profile of amino acid chromatogram of carboxypeptidase Y digested sulfated peptide(A) described in example 2.
- Fig. 5: shows construction method of expression vector for hirudin HV-17 described in example 9.
- Fig. 6: shows profile of HPLC analysis chromatogram of sulfated hirudin HV-17 described in example 10.

In the figure, A and B stands for peptide(A) and peptide(B), respectively.

### Best mode for Carrying Out the Invention

### Example 1

Production of (A) Gly - Asp - Phe - Glu - Glu - Ile - Pro - Glu - Tyr - Tyr - Leu - Gln, and (B) Gly - Asp - Phe - Glu - Glu - Ile - Pro - Tyr - Tyr - Tyr - Leu - Gln Above mentioned peptides (A) and (B) were synthesized by solid phase peptide synthesizer 430A of Applied Biosystems. After deprotection of Boc group of the starting material Boc-Gln-OCH₂-PAM resin (0.5mM) by trifluoroacetate, peptide chain elongation was done by condensation of amino acids in order from C-terminal by symmetrical anhydride method. Thus, the following protected peptides (A') and (B'), as shown in the following formula, bound to resin were obtained.

(A') Gly - Asp(OBzl) - Phe - Glu(OBzl) - Glu(OBzl) - Ile - Pro -
Glu(OBzl) - Tyr(Br-Z) - Tyr(Br-Z) - Leu - Gln - OCH2 - resin

(B') Gly - Asp(OBzl) - Phe - Glu(OBzl) - Glu(OBzl) - Ile - Pro -
Tyr(Br-Z) - Tyr(Br-Z) - Tyr(Br-Z) - Leu - Gln - OCH2 - resin

0.75g of both resins having the protected peptide bound were treated with 17 ml of hydrogen fluoride anhydride in the presence of 1.9 ml of anisole at 0 °C for 1 hour to completely deprotect all the protection groups. After the deprotection, hydrogen fluoride was vaporized off, followed by washing of the residue with diethyl ether and drying with nitrogen gas. After dissolving in 100 ml of 1N acetic acid and removing the resin by filtration, they were loaded on anion exchange column (DOWEX 1-X2) followed by elution of peptides by acetic acid. Next, they were purified by high performance liquid chromatography (HPLC) with following condition.
- Equipment:: Waters Delta Prep 3000
- Column:: Preppack C₁₈, 300 Å
- Running buffer:: A. 0.05% trifluoroacetate / water
B. acetonitrile
- Gradient:: B. 0- 100 % / 100 min.
- Flow rate:: 80 ml / min.
- Detection:: 214 nm

Elution time of peptide (A) was 32 minutes and of peptide (B) was 31 minutes. Then, after removing acetonitrile in the process of concentration, lyophilization was done. Here, the following is the result of amino acid analysis of (A) and (B), respectively, when these peptides were hydrolysed in 6N hydrochloric acid at 110 °C for 24 hours.

| amino acid | (A) | (B) |
|---|---|---|
| Asx | 1.03 (1) | 1.01 (1) |
| Glx | 4.37 (4) | 3.24 (3) |
| Gly | 1.00 (1) | 1.00 (1) |
| Ile | 1.00 (1) | 0.99 (1) |
| Leu | 1.06 (1) | 1.05 (1) |
| Tyr | 2.07 (2) | 3.09 (3) |
| Phe | 1.03 (1) | 1.02 (1) |
| Pro | 1.02 (1) | 1.01 (1) |
| Figures in parenthesis show the theoretical value | | |

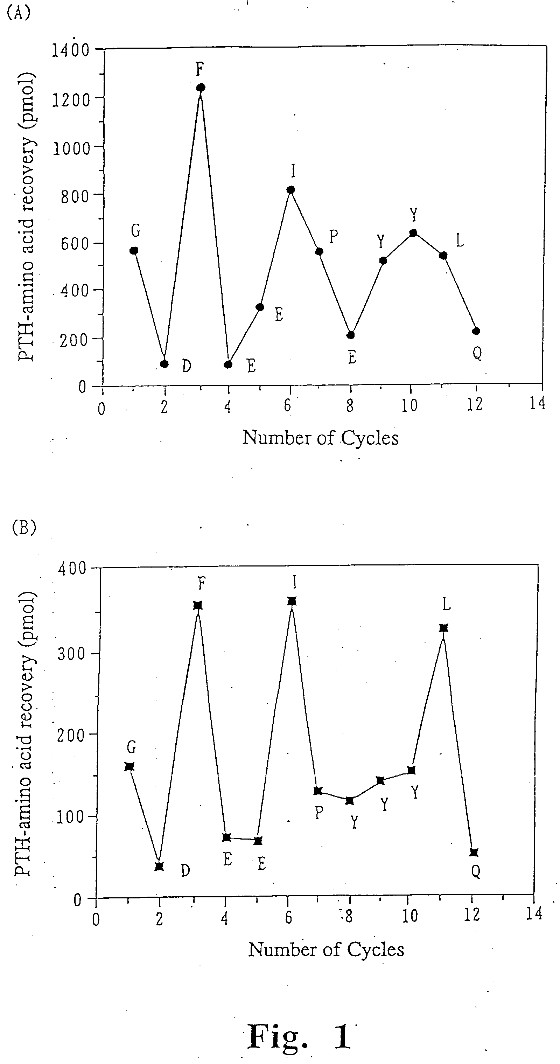

The sequence of aforementioned peptides (A) and (B) were confirmed by gas phase sequencer (Applied Biosystems 477 A). The relation of identified amino acid and recovery of phenylthiohydantoin (PTH) derivative is shown in Fig. 1.

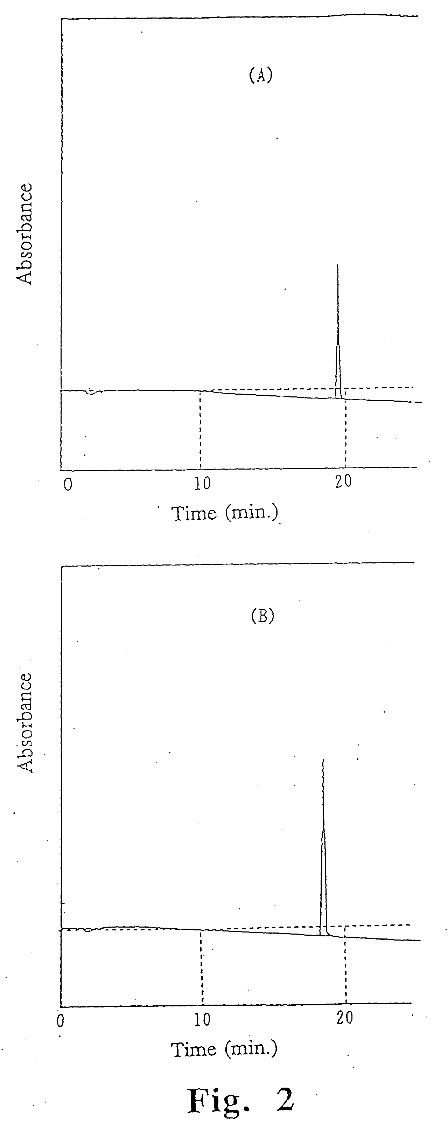

The purity of aforementioned peptides (A) and (B) were both over 99% when analyzed by HPLC [Waters, µ-Bondapak C18 (3.9 x 150 mm)]. The profiles of HPLC analysis chromatogram of each peptide are shown in Fig. 2.

### Example 2

### Production of sulfated form of

(A) Gly - Asp - Phe - Glu - Glu - Ile - Pro - Glu - Tyr - Tyr - Leu - Gln,

and

(B) Gly - Asp - Phe - Glu - Glu - Ile - Pro - Tyr - Tyr - Tyr - Leu - Gln

Sulfation of peptides (A) and (B) synthesized in Example 1 were done using aryl sulfotransferase, derived from human enterobacterium under following condition.

| | |
|---|---|
| Peptide: | 0.1 mM |
| p-nitrophenvlsulfate : | 1.0 mM |
| Sulfotransferase: | 10 U/ml |
| Magnesium chloride : | 25 mM |
| Reaction buffer: | 0.1 M Tris-hydrochloride buffer (pH 8.6) |
| Reaction temperature: | 37 °C |
| Reaction time: | 66 - 96 hours |

Separation and collection of sulfated compounds were done by HPLC under following condition.
- Column:: Waters µ-Bondapak C₁₈ (3.9 x 150 mm)
- Running buffer::
- A. 0.1% trifluoro acetate / water
B. acetonitrile
- Gradient:: B. 10 - 60% / 50 min.
- Flow rate:: 1.5 ml/min.
- Detection:: 230 nm

Under this condition, sulfated peptide (A) eluted at 23.3 minutes, and three kinds of sulfated peptide (B) eluted at 21.4 minutes, 23.4 minutes and 24.6 minutes respectively, Each fraction of sulfated compound was lyophilized after removing acetonitrile in the process of concentration.

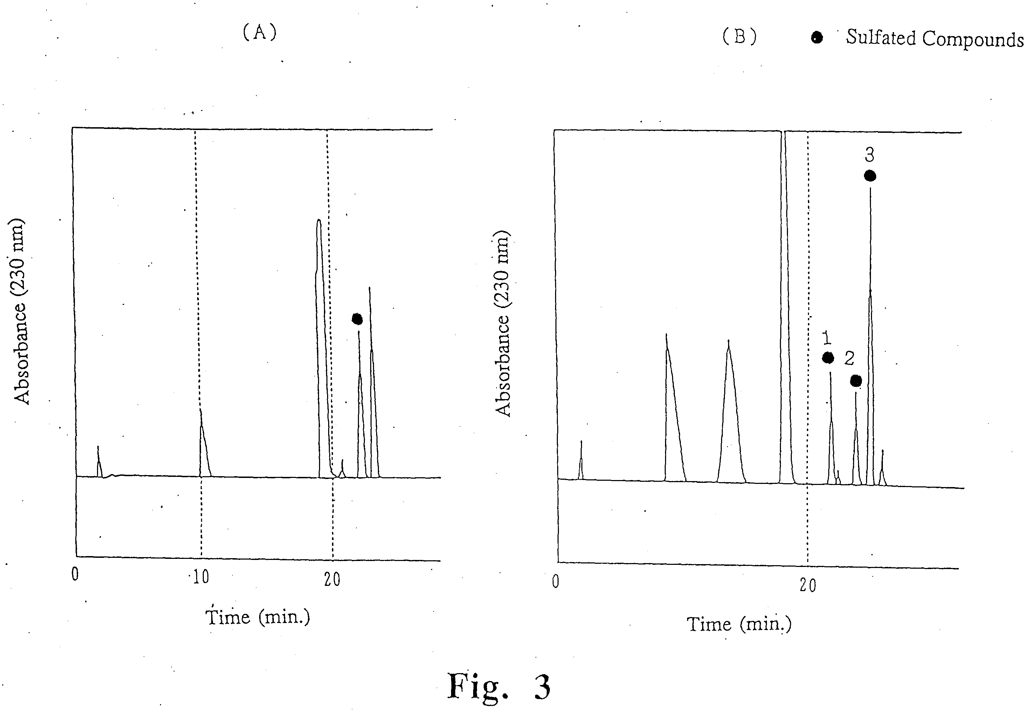

Profiles of HPLC analysis chromatogram of each peptide, sulfated enzymatically under the abovementioned condition, are shown in Fig. 3 (A) and (B), respectively.

### Identification of sulfated site

The identification of sulfated site of aforesaid sulfated peptides (A) and (B) were done by using aminopeptidase M, carboxypeptidase Y, chymotrypsin and V8 protease.

### (1) Identification of sulfated site of peptide (A)

### a. Amino acid analysis after aminopeptidase M digestion

To 10 µl of 1 mM substrate, 5 µl (250 ng) of α-chymotrypsin (product of Sigma, TLCK treated) was added under icy cold condition, followed by 4 hours digestion in 0.1 M sodium-phoshate buffer (pH7.0) at the temperature of 37°C. To this reaction mixture, 5 µl of aminopeptidase M (product of Pierce, 5 mg/ml) was added and additional 18 hours hydrolysis was carried out. Sulfated peptide (A) was confirmed to be mono-sulfated compound by the values of amino acid composition analysis after the hydrolysis.

### b. Digestion by carboxypeptidase Y

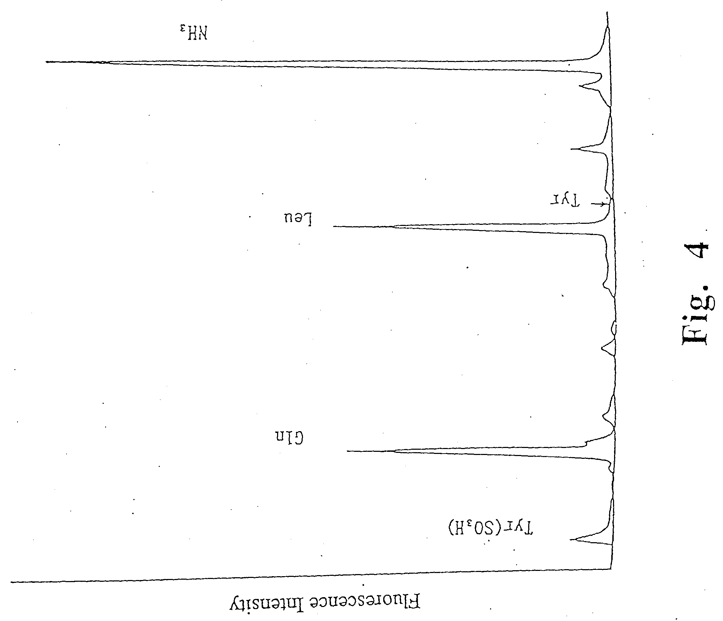

To 10 µl of 1 mM substrate, 1 mg/ml carboxypeptidase Y (product of Boehringer Mannheim) was added under icy cold condition, followed by 30 min. digestion in 0.1 M sodium-phoshate buffer (pH7.0) at the temperature of 37°C. After the digestion, analysis was done using amino acid analyzer (o-phthal aldehyde method). By the chromatogram in Fig. 4, it was confirmed that amino acids Gln, Leu, Tyr(SO₃H) were released from C-terminal side of peptide (A), and no Tyr was released.

From the results in a. and b. above, the sulfated peptide (A) was confirmed to be a mono-sulfated compound of which C-terminal side Tyr was sulfated.

### (2) Identification of sulfated site of peptide (B)

### a. Amino acid analysis after aminopeptidase M digestion

Three kinds of sulfated peptide (B) (peaks 1 - 3 in Fig. 3) were hydrolysed by using aminopeptidase M as described in (1)-a. From the values of amino acid analysis after the hydrolysis, the peak 1 was confirmed to be a disulfated compound, peak 2 and 3 were confirmed to be monosulfated compounds.

### b. Peptide mapping by chymotrypsin digestion

To 10 µl of 1 mM substrate, 5 µl (250 ng) of α-chymotrypsin was added under icy cold condition, followed by 24 hours digestion in 0.1 M sodium-phoshate buffer (pH7.0) at the temperature of 37°C. This reaction mixture was loaded on reverse phase HPLC having Nucleosil 5C₁₈ (4 x 150 mm) as stationary phase, and elution points of the digested peptides were confirmed. As the result, when the substrate was non-sulfated peptide (B), 2 fragments composed of 8 amino acids and 4 amino acids were generated by cleavage of amide bond after the Tyr on N-terminal side. Similarly, three kinds' of sulfated compounds of peaks 1 - 3 were treated in the same way, and in all three cases, the elution point of fragment composed of 8 amino acids were identical to that of non-sulfated peptide. This result shows that in all three kinds of sulfated peptides, Tyr of N-terminal side was not sulfated. Therefore, putting this result and the result in a. together, peak 1 was confirmed to be disulfated compound having two sulfated Tyr residues on C-terminal side.

### c. Digestion by V8 protease

To 10 µl of 1 mM substrate, 5 µl (2.5 unit) of V8 protease (product of Sigma) was added under icy cold condition, followed by 1 hour digestion in 0.1 M sodium-phoshate buffer (pH7.0) at the temperature of 37°C. This reaction mixture was loaded on reverse phase HPLC having Nucleosil 5C₁₈ (4 x 150 mm) as stationary phase, and elution points of the digested peptides were confirmed.

As the result of being sulfated peptide, two fragments composed of eight amino acids and four amino acids were generated from the peptide (A) as the substrate by cleavage of amide bond between Glu and Tyr on N-terminal side. Among them, the elution point of fragment composed of 4 amino acids, that is Tyr-Tyr(SO₃H)-Leu-Gln, was found to be identical with the elution point of fragment composed of 4 amino acids generated by digestion of the peak 3, which is one of the fragments generated by chymotrypsin digestion of peaks 1 - 3 in above b.. From this result, peak 3 was confirmed to be monosulfated compound having Tyr on C-terminal side sulfated. And since the peak 2 having different elution point was shown to be monosulfated compound like peak 3 from above a., it was identified to be - Tyr-Tyr(SO₃H)-Tyr. Above results in (1) and (2) on identification of sulfation site are summarized below.

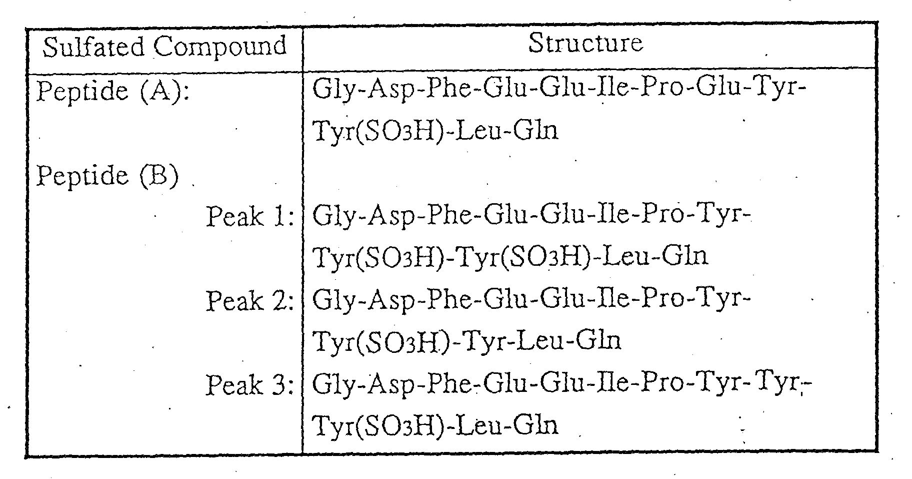

### Example 3

### Production of

(C) Suc-Gly-Asp-Phe-Glu-Pro-Ile-Pro-Glu-Tyr-Tyr-Leu - Gln - OH,
(D) Suc-Gly-Asp-Phe-Glu-Pro-Ile-Pro-Glu-Tyr-Tyr-Leu - Gln - NH2,
(E) Suc-Gly-Asp-Phe-Glu-Pro-Ile-Pro-Tyr-Tyr-Tyr-Leu - Gln - OH,
(F) Suc-Asp-Phe-Glu-Pro-Ile-Pro-Glu-Tyr-Tyr-Leu - Gln - OH,
(G) Suc-Phe-Glu-Pro-Ile-Pro-Glu-Tyr-Tyr-Leu - Gln - OH,

and

(H) Suc-Glu-Pro-Ile-Pro-Glu-Tyr-Tyr-Leu - Gln - OH

Boc-Gln-OCH₂-PAM resin (0.5 mM) was used as the starting material for abovementioned peptides (C), (E), (F), (G) and (H), and p-methyl BHA resin (0.5 mM) for (D). The synthesis was done according to the method described in Example 1, and after the peptide chain elongation, succinylization of N-terminal was done with succinic acid anhydride to obtain protected peptides bound to resin. The peptides bound to resin were deprotected and purified according to the method described in Example 1 to yield abovementioned peptides (C), (D), (E), (F), (G) and (H). Thus obtained peptides were hydrolysed with 6N hydrochloric acid at 110 °C for 24 hours followed by amino acid analysis, and the result in the following table showed the peptides to be aforementioned aimed peptides.

| Amino Acid | (C) | (D) | (E) | (F) | (G) | (H) |
|---|---|---|---|---|---|---|
| Asx | 1.01 (1) | 0.97 (1) | 1.01 (1) | 1.01 (1) | | |
| Glx | 3.25 (3) | 2.70 (3) | 2.13 (2) | 3.06 (3) | 3.05 (3) | 3.06 (3) |
| Gly | 1.00 (1) | 1.00 (1) | 1.00 (1) | | | |
| Ile | 1.00 (1) | 0.92 (1) | 0.95 (1) | 0.97 (1) | 0.98 (1) | 0.98 (1) |
| Leu | 1.05 (1) | 0.96 (1) | 1.01 (1) | 1.03 (1) | 1.04 (1) | 1.03 (1) |
| Tyr | 2.07 (2) | 1.86 (2) | 2.91 (3) | 1.97 (2) | 1.98 (2) | 1.97 (2) |
| Phe | 1.02 (1) | 0.95 (1) | 0.98 (1) | 1.01 (1) | 1.00 (1) | |
| Pro | 1.71 (2) | 1.75 (2) | 1.93 (2) | 1.96 (2) | 1.95 (2) | 1.95 (2) |
| Figures in parenthesis show the theoretical value | | | | | | |

### Example 4

### Production of sulfated form of

(C) Suc-Gly-Asp-Phe-Glu-Pro-Ile-Pro-Glu-Tyr-Tyr-Leu - Gln - OH,
(D) Suc-Gly-Asp-Phe-Glu-Pro-Ile-Pro-Glu-Tyr-Tyr-Leu - Gln - NH2,
(E) Suc-Gly-Asp-Phe-Glu-Pro-Ile-Pro-Tyr-Tyr-Tyr-Leu - Gln - OH,
(F) Suc-Asp-Phe-Glu-Pro-Ile-Pro-Glu-Tyr-Tyr-Leu - Gln - OH,
(G) Suc-Phe-Glu-Pro-Ile-Pro-Glu-Tyr-Tyr-Leu - Gln - OH,

and

(H) Suc-Glu-Pro-Ile-Pro-Glu-Tyr-Tyr-Leu - Gln - OH

Each of 50 mg of peptides (C), (D), (F), (G) and (H) produced in Example 3 were dissolved in 4 ml of 20% pyridine / dimethylformamide, followed by sulfation by addition of 1.05 g (225 equivalent) of pyridine-sulfur trioxide under room temperature (25 °C), and 4 hours of reaction time.

On the other hand, peptide (E) was sulfated by condensation method using dicyclohexylcarbodiimide (DCC). That is, to 1 µmol of this peptide, 10 µl of dimethylformamide containing 40 µmol of concentrated sulfuric acid was added, and sulfation was carried out at room temperature (25°C) for 1 minute with stirring.

Sulfated compounds obtained by aforementioned process were purified using reverse phase HPLC under the following condition.
- Equipment:: Shimazu LC-6A
- Column:: Waters µ-Bondapak C₁₈ (3.9 x 300 mm)
- Running Buffer:: 0.1% trifluoro acetate, acetonitrile
- Gradient:: acetonitrile 10 - 60% / 50 min.
- Flow rate:: 1.5 ml min.
- Detection:: 230 nm

Under this condition, sulfated peptide (D) eluted at 18.7 minutes, sulfated (E) at 17.6 minutes, sulfated (F) at 18.0 minutes, sulfated (G) at 19.3 minutes and sulfated (H) at 13,6 minutes, respectively. Each sulfated compound was collected, concentrated, desalted by gel filtration (product of Pharmacia, Sephadex G-10), pH adjusted at 7.0 - 7.5 with 10% aqueous ammonia and lyophilized. By this method, stable sulfated compounds were recovered as ammonium salts.

### Identification of sulfation site

According to the method of identification of sulfated site described in Example 2, sulfated sites of abovementioned sulfated peptides were identified. As the result, structure of each sulfated compound became clear as shown below.

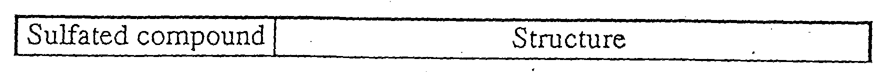

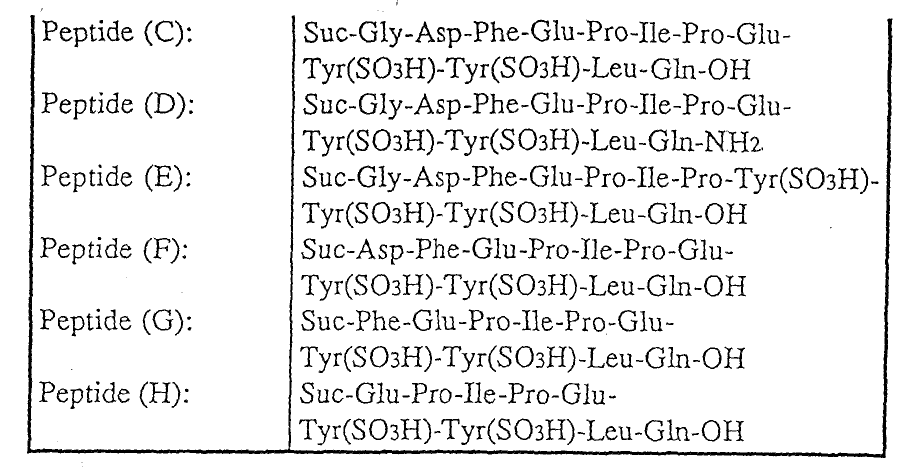

### Example 5

### Production of

(I) Suc-Phe-Glu-Pro-Ile-Pro-Glu-Tyr-Tyr-OH,
(J) Suc-Phe-Glu-Pro-Ile-Pro-Glu-Tyr-Tyr-NH2,
(K) Suc-Phe-Glu-Pro-Ile-Pro-Glu-Tyr-Tyr-NH(CH2)2COOH,
(L) Suc-Phe-Glu-Pro-Ile-Pro-Glu-Tyr-Tyr-NH(CH2)4COOH,
(M) Suc-Phe-Glu-Pro-Ile-Pro-Glu-Tyr-Tyr-Tau-NH2,
(N) Suc-Phe-Glu-Pro-Ile-Pro-Glu-Tyr-Tyr-NH(CH2)3OH,

and

(O) Suc-Phe-Glu-Pro-Ile-Pro-Glu-Tyr-Tyr-(L)Asu(OMe)OMe,

As the starting material used for all abovementioned peptides (I), (J), (K), (L), (M), (N) and (O), the protected peptide shown in the following formula (i) was first produced by liquid phase method. 
(i) Boc-Phe-Glu(OBzl)-Pro-Ile-Pro-Glu(OBzl)-Tyr(Bzl-Cl2)-
Tyr(Bzl-Cl2)-OH
 Peptide (i) was obtained by the following procedures. After Boc group was removed from the starting material Boc-Tyr(Bzl-Cl₂)OPac (OPac stands for phenacyl ester) with trifluoroacetate, condensation was done with Boc-Tyr(Bzl-Cl₂)-OH by DCC-HOBt method and Boc-Tyr(Bzl-Cl₂)-Tyr(Bzl-C1₂)-OPac was obtained. Then, starting from Boc-Tyr(Bzl-Cl₂)-Tyr(Bzl-Cl₂)-OPac, according to the aforementioned method, elongation of peptide was done by repeated deprotection and condensation of protected amino acids in order, and the protected peptide shown in the following formula (ii) was obtained.

(ii)	Boc-Phe-Glu(OBzl)-Pro-Ile-Pro-Glu(OBzl)-Tyr(Bzl-Cl2)-
Tyr(Bzl-Cl2)-OPac

After peptide (ii) was dissolved in acetate in water dipped container, zinc powder was added to it, stirred for 2 hours, removed the zinc powder from the reaction mixture, concentrated under vacuum pressure and abovementioned peptide (i) was obtained.

Abovementioned peptide (I) was synthesized by the following procedure using peptide (i) as starting material.

First, Boc group was removed from peptide (i) with trifluoroacetate; followed by treating it with succinic acid anhydride in the presence of base and the protected peptide (iii) shown in the formula below was obtained.

(iii) Suc-Phe-Glu(OBzl)-Pro-Ile-Pro-Glu(OBzl)-Tyr(Bzl-Cl2)-
Tyr(Bzl-Cl2)-OH

Peptide (iii) was treated with hydrogenfluoride at 0° C for 1 hour in the presence of anisole to remove all the protecting groups. After the removal, hydrogenfluoride was removed in vacuum pressure, residue was washed with diethylether and dissolved in 1N acetic acid, charged on highly basic ion exchange column (Diaion PA-308), and peptide was eluted by 50% acetic acid.

Then, purification was done by gel filtration under following condition.
- Column:: Sephadex LH-20 (2.2 φ x 97 cm)
- Running buffer:: 50% MeOH/H₂O
- Flow rate:: 0.8 ml/min.
- Detection:: UV 230 nm, 280 nm

Appropriate portion was collected and lyophilized to obtain the aimed compound (I).

Abovementioned peptide (J) was synthesized by the following procedure using peptide (i) as starting material.

First, DCC-HOSu was reacted with chloroform solution of peptide (i) to convert peptide (i) to activated ester. To this solution, ammonium gas was introduced, followed by vacuum concentration, removal of Boc group from obtained residue with procedure similar to the synthesis of (I), succinylization, and protected peptide (iv) shown in the formula below was obtained. 
(iv) Suc-Phe-Glu(OBzl)-Pro-Ile-Pro-Glu(OBzl)-Tyr(Bzl-Cl2)-
Tyr(Bzl-Cl2)-NH2
 Peptide (iv) was treated with hydrogenfluoride at 0 °C for 1 hour in the presence of anisole to remove all the protecting groups. After the removal, hydrogen fluoride was removed in vacuum pressure, residue was washed with diethylether and dissolved in 1N acetic acid, charged on highly basic ion exchange column, and peptide was eluted by 50% acetic acid. Then, purification was done by gel filtration under following condition. Appropriate portion was collected and lyophilized to obtain the aimed compound (J).

Abovementioned peptide (K) was synthesized by the following procedure using peptide (i) as starting material.

First, condensation of peptide (i) and NH₂(CH₂)₂COOBzl·HCl was done by DCC-HOBt method and following protected peptide (v) was obtained.

(v) Boc-Phe-Glu(OBzl)-Pro-Ile-Pro-Glu(OBzl)-Tyr(Bzl-Cl2)-
Tyr(Bzl-Cl2)-NH(CH2)2COOBzl

Boc group of peptide (v) was removed by the method similar to the one described in abovementioned synthesis of (I), followed by succinylization of N-terminal end, deprotection of obtained protected peptide by the method similar to the one described in abovementioned synthesis of (I), purification, and the aimed compound (K) was obtained.

Abovementioned peptide (L) was synthesized by the following procedure using peptide (i) as starting material.

First, using peptide (i) and NH₂(CH₂)₄COOBzl·HCl, following protected peptide (vi) was obtained by the method similar to abovementioned peptide (K).

(vi) Boc-Phe-Glu(OBzl)-Pro-Ile-Pro-Glu(OBzl)-Tyr(Bzl-Cl2)-
Tyr(Bzl-Cl2)-NH(CH2)4COOBzl

By the method similar to abovementioned peptide (K), N-terminal end of peptide (vi) was succinylized, followed by deprotection of obtained protected peptide by the method similar to the one described in abovementioned synthesis of (K), purification, and the aimed compound (L) was obtained.

Abovementioned peptide (M) was synthesized by the following procedure using peptide (i) as starting material.

First, using peptide (i) and Tau-NH₂·HCl, following protected peptide
(vii) was obtained by the method similar to abovementioned peptide (K).
(vii) Boc-Phe-Glu(OBzl)-Pro-Ile-Pro-Glu(OBzl)-Tyr(Bzl-Cl₂)-Tyr(Bzl-Cl₂)-Tau-NH₂

By the method similar to abovementioned peptide (K), N-terminal end of peptide (vii) was succinylized, followed by deprotection of obtained protected peptide by the method similar to the one described in abovementioned synthesis of (K), purification, and the aimed compound (M) was obtained.

Abovementioned peptide (N) was synthesized by the following procedure using peptide (i) as starting material.

First, using peptide (i) and H₂N(CH₂)₃OH, following protected peptide (viii) was obtained by the method similar to abovementioned peptide (K).

(viii) Boc-Phe-Glu(OBzl)-Pro-Ile-Pro-Glu(OBzl)-Tyr(Bzl-Cl2)-
Tyr(Bzl-Cl2)-NH(CH2)3OH

By the method similar to abovementioned peptide (K), N-terminal end of peptide (viii) was succinylized, followed by deprotection of obtained protected peptide by the method similar to the one described in abovementioned synthesis of (K), purification, and the aimed compound (N) was obtained.

Abovementioned peptide (O) was synthesized by the following procedure using peptide (i) as starting material.

First, using peptide (i) and (L)Asu(OMe)OMe·HCl, following protected peptide (ix) was obtained by the method similar to abovementioned peptide (K).

(ix) Boc-Phe-Glu(OBzl)-Pro-Ile-Pro-Glu(OBzl)-Tyr(Bzl-Cl2)-
Tyr(Bzl-Cl2)-(L)Asu(OMe)OMe

By the method similar to abovementioned peptide (K), N-terminal end of peptide (ix) was succinylized, followed by deprotection of obtained protected peptide by the method similar to the one described in abovementioned synthesis of (K), purification, and the aimed compound (O) was obtained.

Followings are results of amino acid analysis value after hydrolysis of these peptides in 6N hydrochloride at 110 °C for 24 hours, and Rf value of thin layer chromatography. The results show the abovementioned aimed peptides were obtained.

| Amino Acid | (I) | (J) | (K) | (L) | (M) | (N) | (O) |
|---|---|---|---|---|---|---|---|
| Glu | 2.17(2) | 2.13(2) | 2.12(2) | 2.14(2) | 2.15(2) | 2.19(2) | 2.15(2) |
| Pro | 2.08(2) | 1.89(2) | 2.00(2) | 1.83(2) | 1.84(2) | 1.83(2) | 2.09(2) |
| Ile | 0.98(1) | 0.96(1) | 0.91(1) | 0.95(1) | 0.99(1) | 1.00(1) | 0.93(1) |
| Tyr | 1.92(2) | 2.12(2) | 2.01(2) | 2.11(2) | 2.13(2) | 2.15(2) | 2.11(2) |
| Phe | 1.00(1) | 1.00(1) | | 1.00(1) | 1.00(1) | 1.00(1) | 1.00(1) |
| Tau-NH₂ | | | | 1.11(1) | | | |
| Asu | | | | | | | 0.98(1) |
| NH₂(CH₂)₂COOH+Phe | | | 1.93(2) | | | | |
| NH₂(CH₂)₄COOH | | | | 0.89(1) | | | |
| Figures in parenthesis show the theoretical value | | | | | | | |

| TLC | (I) | (J) | (K) | (L) | (M) | (N) | (O) |
|---|---|---|---|---|---|---|---|
| I | 0.63 | 0.71 | 0.71 | 0.66 | 0.68 | 0.72 | 0.84 |
| II | 0.54 | 0.59 | 0.55 | 0.52 | 0.59 | 0.59 | 0.64 |

Merck 20 x 20 silica gel 60 glass plates F₂₅₄, 0.25 mm thickness

I; CHCl₃ / MeOH / AcOH (5/2/1) II; n-BuOH / AcOH / H₂O / Pyridine (15/3/12/10)

### Example 6

### Production of sulfated form of

(I) Suc-Phe-Glu-Pro-Ile-Pro-Glu-Tyr-Tyr-OH,
(J) Suc-Phe-Glu-Pro-Ile-Pro-Glu-Tyr-Tyr-NH2,
(K) Suc-Phe-Glu-Pro-Ile-Pro-Glu-Tyr-Tyr-NH(CH2)2COOH,
(L) Suc-Phe-Glu-Pro-Ile-Pro-Glu-Tyr-Tyr-NH(CH2)4COOH,
(M) Suc-Phe-Glu-Pro-Ile-Pro-Glu-Tyr-Tyr-Tau-NH2,
(N) Suc-Phe-Glu-Pro-Ile-Pro-Glu-Tyr-Tyr-NH(CH2)3OH,

and

(O) Suc-Phe-Glu-Pro-Ile-Pro-Glu-Tyr-Tyr-(L)Asu(OMe)OMe,

Each of 50mg of peptides (I), (J), (K), (L), (M), (N) and (O), produced in Example 5, were dissolved in 20 ml of pyridine/dimethylformamide (1:1), 1.29 g (200 equivalents) of pyridine-sulfur trioxide was added at room temperature (25 °C), and 4 hours reaction was carried out for sulfation. Then the sulfated compounds were controlled at pH7.0 with aqueous and saturated solution of sodium hydrogen carbonate, precipitates were filtered and filtrates were concentrated under reduced pressure.

The aforementioned sulfated compounds were purified by reverse phase HPLC under following condition.
- Equipment:: Nihon Bunkou 808-SC
- Column:: Waters µ-Bondapak 5C₁₈ (3.9 x 300 mm)
- Running Buffer:: Methanol, 10 mM ammonium acetate aqueous solution (pH 6.0)
- Gradient:: Methanol 1→60% / 60 min.
- Flow rate:: 1 ml/min.
- Detection:: 230 nm and 280 nm

Under this condition, each sulfated compound was collected and lyophilized, and the obtained powder was dissolved in 0.05N acetic acid and desalted by Sephadex G-10. Appropriate portions were collected and lyophilized to obtain the aimed sulfated compounds as sodium salts.

### Identification of sulfation site

The sites of sulfation of abovementioned sulfated peptides were identified according to the method of identification of sulfation site described in Example 2. As the result, structure of each sulfated compound became clear as shown below.

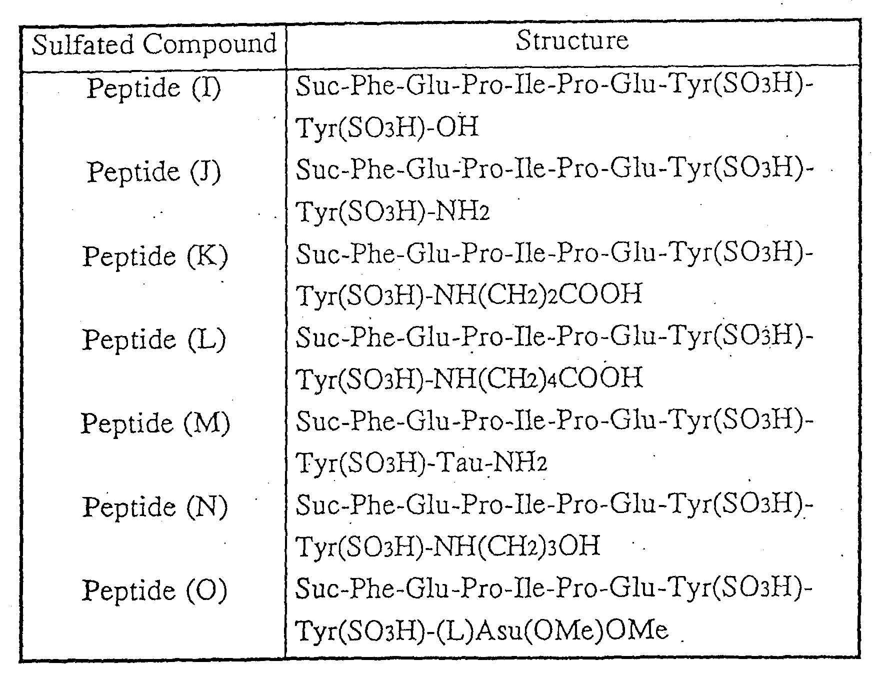

### Example 7

### Production of

(P) Suc-Phe-Glu-Pro-Ile-Pro-Glu-Tyr-Tyr-Leu-ol,
(Q) Suc-Phe-Glu-Pro-Ile-Pro-Glu-Tyr-Tyr-Leu-OH,
(R) Suc-Phe-Glu-Pro-Ile-Pro-Glu-Tyr-Tyr-Leu-NHEt,
(S) Suc-Phe-Glu-Pro-Ile-Pro-Glu-Tyr-Tyr-Leu-Tau-NH2,
(T) Suc-Phe-Glu-Pro-Ile-Pro-Glu-Tyr-Tyr-Leu-(D)Glu-OH,
(U) Suc-Phe-Glu-Pro-Ile-Pro-Glu-Tyr-Tyr-Leu-(D)Asu(OMe)OMe,
(V) Suc-Phe-Glu-Pro-Ile-Pro-Glu-Tyr-Tyr-Leu-(L)Asu(OMe)OMe,
(W) Suc-Phe-Glu-Pro-Ile-Pro-Glu-Tyr-Tyr-Leu-(D)Asu(NH2)NH2,
(X) Suc-Phe-Glu-Pro-Ile-Pro-Glu-Tyr-Tyr-Leu-(L)Asu(NH2)NH2,

and

(Y) Suc-Phe-Glu-Pro-Ile-Pro-Glu-Tyr-Tyr-Leu-NHPO(OH)2

As the starting material used for all abovementioned peptides (Q), (R), (S), (T), (U), (V), (W) and (X), the protected peptide shown in the following formula (x) was first produced by liquid phase method.

(x) Boc-Phe-Glu(OBzl)-Pro-Ile-Pro-Glu(OBzl)-Tyr(Bzl-Cl2)-
Tyr(Bzl-Cl2)-Leu-OH

Peptide (x) synthesis was done as follows. First, by condensation of protected peptide (i) synthesized in Example 5 and TOsOH·Leu-OTce (OTce stands for 2,2,2-trichloroethylester) was done by WSCI-HOBt method and peptide (xi) shown in the formula below was synthesized.

(xi) Boc-Phe-Glu(OBzl)-Pro-Ile-Pro-Glu(OBzl)-Tyr(Bzl-Cl2)-
Tyr(Bzl-Cl2)-Leu-OTce

Next, after peptide (xi) was dissolved in acetate in water dipped container, zinc powder was added to it, stirred for 2 hours for Tce deprotection, removed the zinc powder from the reaction mixture, concentrated under vacuum pressure and abovementioned peptide (x) was obtained.

Abovementioned peptide (P) was synthesized by the following procedure using peptide (i) as starting material.

First, condensation of peptide (i) and Leu-ol was done by WSCI-HOBt method and protected peptide (xii) shown in the formula below was obtained.

(xii) Boc-Phe-Glu(OBzl)-Pro-Ile-Pro-Glu(OBzl)-Tyr(Bzl-Cl2)-
Tyr(Bzl-Cl2)-Leu-ol

Boc group was removed from peptide (xii) according to the synthesis method of (I), N-terminal end of peptide (xii) was succinylized, followed by deprotection of obtained protected peptide by the method similar to the one described in abovementioned synthesis of (I), purification, and the aimed compound (P) was obtained.

Above mentioned peptide (Q) was synthesized by the following procedure using peptide (x) as starting material.

First, Boc group was removed from peptide (x) with trifluofoacetate, followed by treating it with succinic acid anhydride in the presence of base and the protected peptide (xiii) shown in the following formula was obtained.

(xiii) Suc-Phe-Glu(OBzl)-Pro-Ile-Pro-Glu(OBzl)-Tyr(Bzl-Cl2)-
Tyr(Bzl-Cl2)-Leu-OH

Peptide (xiii) was treated with anhydrous hydrogen fluoride at 0 °C for 1 hour in the presence of anisole to remove all the protecting groups. After the removal, hydrogen fluoride was removed in vacuum pressure, residue was washed with diethylether and dissolved in 1N acetic acid, charged on highly basic ion exchange column, and peptide was eluted by 50% acetic acid. Then, purification was done by gel filtration under the condition described before and aimed compound (Q) was obtained.

Abovementioned peptide (R) was synthesized by the following procedure using peptide (x) as starting material.

First, condensation of peptide (x) and NH₂Et was done by WSCI-HOBt method and protected peptide (xiv) shown in the formula below was obtained.

(xiv) Boc-Phe-Glu(OBzl)-Pro-Ile-Pro-Glu(OBzl)-Tyr(Bzl-Cl2)-
Tyr(Bzl-Cl2)-Leu-NHEt

Boc group was removed from peptide (xiv) according to the synthesis method of (Q), followed by succinylization of N-terminal, deprotection of obtained protected peptide according to the synthesis method of (Q), purification, and aimed compound (R) was obtained.

Abovementioned peptide (S) was synthesized by the following procedure using peptide (x) as starting material.

First, protected peptide (xv) shown in the formula below was obtained using peptide (x) and Tau-NH2·HCl with method similar to the peptide (M).

(xv) Boc-Phe-Glu(OBzl)-Pro-Ile-Pro-Glu(OBzl)-Tyr(Bzl-Cl2)-
Tyr(Bzl-Cl2)-Leu-Tau-NH2

Succinylization of N-terminal of peptide (xv) was done with method similar to peptide (M), deprotection of obtained protected peptide according to the synthesis method of (M), and after the purification, aimed compound (S) was obtained.

Abovementioned peptide (T) was synthesized by the following procedure using peptide (x) as starting material.

First, protected peptide (xvi) shown in the formula below was obtained using peptide (x) and (D)Glu(OBzl)OBzl·HCl with method similar to the peptide (S).

(xvi) Boc-Phe-Glu(OBzl)-Pro-Ile-Pro-Glu(OBzl)-Tyr(Bzl-Cl2)-
Tyr(Bzl-Cl2)-Leu-(D)Glu(OBzl)OBzl

Succinylization of N-terminal of peptide (xvi) was done with method similar to peptide (S), deprotection of obtained protected peptide according to the synthesis method of (S), and after the purification, aimed compound (T) was obtained.

Abovementioned peptide (U) was synthesized by the following procedure using peptide (x) as starting material.

First, protected peptide (xvii) shown in the formula below was obtained using peptide (x) and (D)Asu(OMe)OMe·HCl with method similar to the peptide (S).

(xvii) Boc-Phe-Glu(OBzl)-Pro-Ile-Pro-Glu(OBzl)-Tyr(Bzl-Cl2)-
Tyr(Bzl-Cl2)-Leu-(D)Asu(OMe)OMe

Succinylization of N-terminal of peptide (xvii) was done with method similar to peptide (S), deprotection of obtained protected peptide according to the synthesis method of (S), and after the purification, aimed compound (U) was obtained.

Abovementioned peptide (V) was synthesized by the following procedure using peptide (x) as starting material.

First, protected peptide (xviii) shown in the formula below was obtained using peptide (x) and (L)Asu(OMe)OMe·HCl with method similar to the peptide (S).

(xviii) Boc-Phe-Glu(OBzl)-Pro-Ile-Pro-Glu(OBzl)-Tyr(Bzl-Cl2)-
Tyr(Bzl-Cl2)-Leu-(L)Asu(OMe)OMe

Succinylization of N-terminal of peptide (xviii) was done with method similar to peptide (S), deprotection of obtained protected peptide according to the synthesis method of (S), and after the purification, aimed compound (V) was obtained.

Abovementioned peptide (W) was synthesized by the following procedure using peptide (x) as starting material.

First, protected peptide (xix) shown in the formula below was obtained using peptide (x) and (D)Asu(NH₂)NH₂·HCl with method similar to the peptide (S).

(xix) Boc-Phe-Glu(OBzl)-Pro-Ile-Pro-Glu(OBzl)-Tyr(Bzl-Cl2)-
Tyr(Bzl-Cl2)-Leu-(D)Asu(NH2)NH2

Succinylization of N-terminal of peptide (xix) was done with method similar to peptide (S), deprotection of obtained protected peptide according to the synthesis method of (S), and after the purification, aimed compound (W) was obtained.

Abovementioned peptide (X) was synthesized by the following procedure using peptide (x) as starting material.

First, protected peptide (xx) shown in th formula below was obtained using peptide (x) and (L)Asu(NH₂)NH₂·HCl with method similar to the peptide (S).

(xx) Boc-Phe-Glu(OBzl)-Pro-Ile-Pro-Glu(OBzl)-Tyr(Bzl-Cl2)-

Tyr(Bzl-Cl2)-Leu-(L)Asu(NH2)NH2

Succinylization of N-terminal of peptide (xx) was done with method similar to peptide (S), deprotection of obtained protected peptide according to the synthesis method of (S), and after the purification, aimed compound (X) was obtained.

Abovementioned peptide (Y) was synthesized by the following procedure using peptide (i) as starting material. First, protected peptide (xxi) shown in the formula below was obtained using peptide (i) and Leu-NHPO(OH)₂·HCl with method similar to the peptide (S).

(xxi) Boc-Phe-Glu(OBzl)-Pro-Ile-Pro-Glu(OBzl)-Tyr(Bzl-Cl2)-
Tyr(Bzl-Cl2)-Leu-NHPO(OH)2

Succinylization of N-terminal of peptide (xxi) was done with method similar to peptide (S), deprotection of obtained protected peptide according to the synthesis method of (S), and after the purification, aimed compound (Y) was obtained.

Followings are results of amino acid analysis value after hydrolysis of these peptides in 6N hydrochloric acid at 110 °C for 24 hours, and Rf value of thin layer chromatography. The results show the abovementioned aimed peptides were obtained.

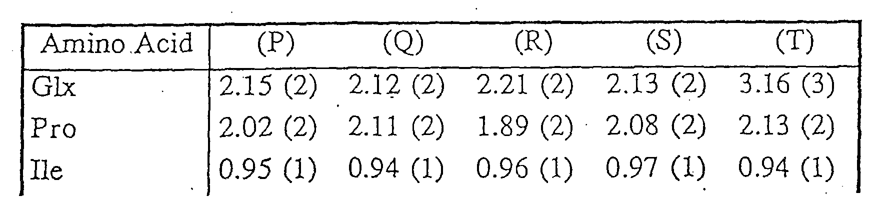

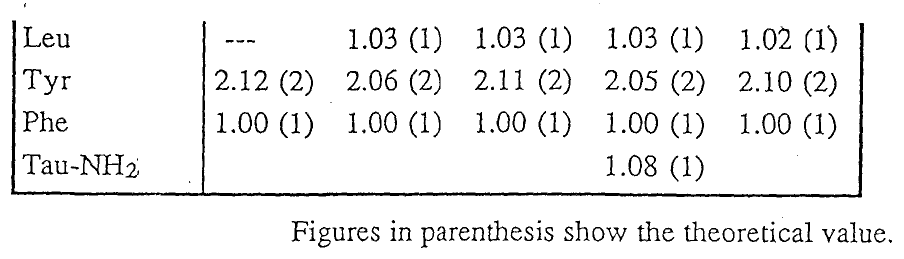

| Amino Acid | (U) | (V) | (W) | (X) | (Y) |
|---|---|---|---|---|---|
| Glx | 2.23 (2) | 2.18 (2) | 2.25 (2) | 2.13 (2) | 2.08 (2) |
| Pro | 1.81 (2) | 2.18 (2) | 1.83 (2) | 2.12 (2) | 1.83 (2) |
| Ile | 0.93 (1) | 0.98 (1) | 0.97 (1) | 0.94 (1) | 0.94 (1) |
| Leu | 0.92 (1) | 0.95 (1) | 1.02 (1) | 1.03 (1) | -- |
| Tyr | 2.13 (2) | 2.09 (2) | 2.13 (2) | 2.01 (2) | 2.19 (2) |
| Phe | 1.00 (1) | 1.00 (1) | 1.00 (1) | 1.00 (1) | 1.00 (1) |
| Asu | 1.06 (1) | 1.10 (1) | 1.12 (1) | 1.09 (1) | |
| Figures in parenthesis show the theoretical value. | | | | | |

| TLC | (P) | (Q) | (R) | (S) | (T) |
|---|---|---|---|---|---|
| I | 0.77 | 0.71 | 0.89 | 0.74 | 0.56 |
| II | 0.60 | 0.57 | 0.62 | 0.62 | 0.48 |

| TLC | (U) | (V) | (W) | (X) | (Y) |
|---|---|---|---|---|---|
| I | 0.93 | 0.85 | 0.75 | 0.71 | |
| II | 0.63 | 0.62 | 0.58 | 0.60 | |

Merck 20 x 20 silica gel 60 glass plates F₂₅₄, 0.25 mm thickness I; CHCl₃ / MeOH / AcOH (5/2/1), II; n-BUOH /AcOH/H₂O/Pyridine (15/3/12/10)

### Example 8

### Production of sulfated form of

(P) Suc-Phe-Glu-Pro-Ile-Pro-Glu-Tyr-Tyr-Leu-ol,
(Q) Suc-Phe-Glu-Pro-Ile-Pro-Glu-Tyr-Tyr-Leu-OH,
(R) Suc-Phe-Glu-Pro-Ile-Pro-Glu-Tyr-Tyr-Leu-NHEt,
(S) Suc-Phe-Glu-Pro-Ile-Pro-Glu-Tyr-Tyr-Leu-Tau-NH2,
(T) Suc-Phe-Glu-Pro-Ile-Pro-Glu-Tyr-Tyr-Leu-(D)Glu-OH,
(U) Suc-Phe-Glu-Pro-Ile-Pro-Glu-Tyr-Tyr-Leu-(D)Asu(OMe)OMe,
(V) Suc-Phe-Glu-Pro-Ile-Pro-Glu-Tyr-Tyr-Leu-(L)Asu(OMe)OMe,
(W) Suc-Phe-Glu-Pro-Ile-Pro-Glu-Tyr-Tyr-Leu-(D)Asu(NH2)NH2,
(X) Suc-Phe-Glu-Pro-Ile-Pro-Glu-Tyr-Tyr-Leu-(L)Asu(NH2)NH2,

and

(Y) Suc-Phe-Glu-Pro-Ile-Pro-Glu-Tyr-Tyr-Leu-NHPO(OH)2

About 50 mg each of peptides (P), (Q), (R), (S), (T), (U), (V), (W), (X) and (Y), produced in Example 7, were dissolved in 20 ml of pyridine/dimethylformamide (1:1), 1.29 g (200 equivalents) of pyridine-sulfur trioxide was added at room temperature (25 °C), and 4 hours reaction was carried out for sulfation. Then, pH was adjusted to 7.0 with saturated sodium hydrogencarbonate solution, followed by removal of precipitates deposited by this process by filtration, and condensation of filtrates under vacuum pressure.

The aforementioned sulfated compounds were purified by reverse phase HPLC under following condition.
- Equipment:: Nihon Bunkou 808-SC
- Column:: Waters µ-Bondapak 5C₁₈ (3.9 x 300 mm)
- Running Buffer:: Methanol, 10 mM ammonium acetate aqueous solution (pH 6.0)
- Gradient:: Methanol 1→60% / 60 min.
- Flow rate:: 1 ml/min.
- Detection:: 230 nm and 280 nm

Under this condition, each sulfated compound was collected and lyophilized, and the powder was dissolved in 0.05N acetic acid and desalted by Sephadex G-10 (product of Pharmacia). Appropriate portions were collected and lyophilized to obtain the aimed sulfated compounds as sodium salts.

### Identification of sulfation site

The sites of sulfation of abovementioned sulfated peptides were identified according to the method of identification of sulfation site described in Example 2. As the result, structure of each sulfated compound became clear as shown below.

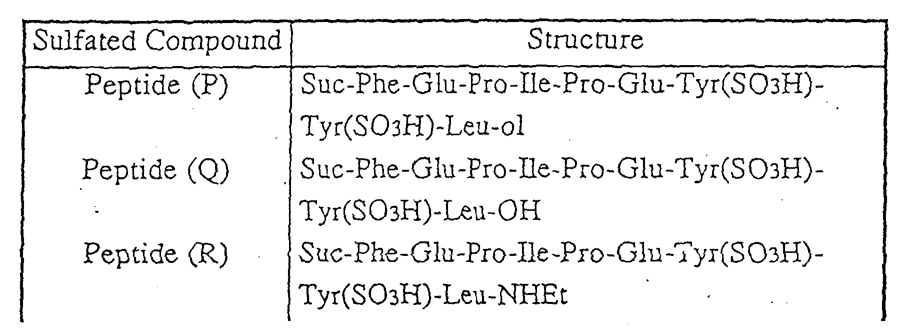

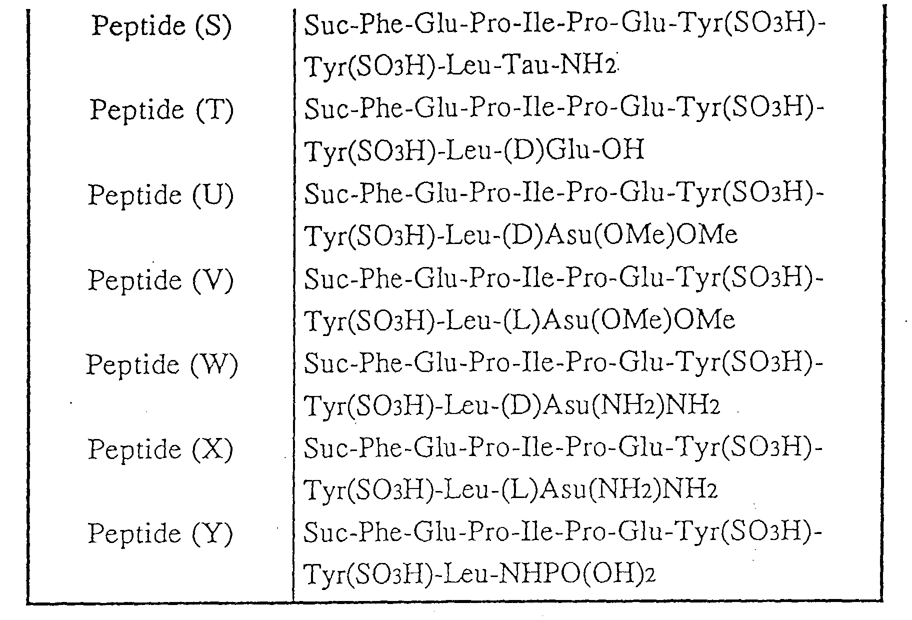

### Example 9

### (1) Method of mutatin Glu 61-62 of hirudin Hv-1 to Tvr 61-62

The mutation primer having DNA sequence shown in formula (III) below was synthesized by method of phosphoamidite using DNA synthesizer (Applied Biosystems 380B).

GAA ATC CCG TAC TAC TAC CTG CAG (III)

1 µg of commercially available double stranded phage M13 mp19 DNA (product of Toyobo) was digested with restriction enzymes EcoRI (10 units) and HindIII (10 units) and purified by agarose gel electrophoresis.

On the other hand, 10 µg of vector pUCHV1 (reference: Japanese Laid-Open Patent 3-164184), in which HV-1 gene was cloned into commercially available plasmid pUC18, was digested with restriction enzymes EcoRI (30 units) and HindIII (30 units) to obtain a 210 bp DNA fragment coding HV-1.

100 ng of this fragment and 100 ng of abovementioned purified EcoRI-HindIII fragment of phage M13 mp19 were ligated at 16 °C for overnight by T4 DNA ligase. Using this reaction mixture, E. coli JM101 was transformed according to the method of J. Messing [ methods in Enzymology, 101, 21-78 (1983)]. From each of obtained plaque, single stranded DNA (M13HV1) was prepared according to the method of J. Messing (the same with above). These were dissolved in TE buffer (pH8.0) at the final concentration of 1 mg/ml.

50 pmol of mutation primer shown in abovementioned formula (III) was phosphorylated in kinase buffer (0.1 M Tris-hydrochloric acid buffer pH8.0, 0.1 mM MgCl₂, 7 mM Dithiothreitol, lmM ATP) containing 2 units of T4 polynucleotidekinase at 37 °C for 15 min, followed by heating at 70 °C for 10 min to terminate the reaction.

Mutated DNA M13HV17 was constructed using 5 µg of aforesaid M13HV1 and 4 pmol of abovementioned phosphorylated mutation oligodeoxyribonucleotide, according to the method of "Oligonucleotide-directed in vitro mutagenesis system" kit, the product of Amersham, which is an applied method of Eckstein et al. [Nucleic Acids Research, 13, 8764 (1985)]

### (2) Preparation of mutated gene and construction of mutated peptide secreting plasmid

20 µl of solution containing mutated DNA M13HV17 obtained in abovementioned (1), competent E. coli cell TG1 was transformed and plaques were obtained. Double strand DNA was prepared from this transformant.

30 µg of abovementioned double strand DNA was digested with restriction enzymes AccI and HindIII, and purified 200 bp DNA fragment having an N-terminal amino acid coding region deleted.

On the other hand, hirudin expression vector pMTSHV1 (Reference: Japanese Laid-Open Patent 2-303096) was digested with restriction enzymes- AccI and HindIII as described above, and purified 2.75 Kb DNA fragment including DNA coding phoA signal peptide.

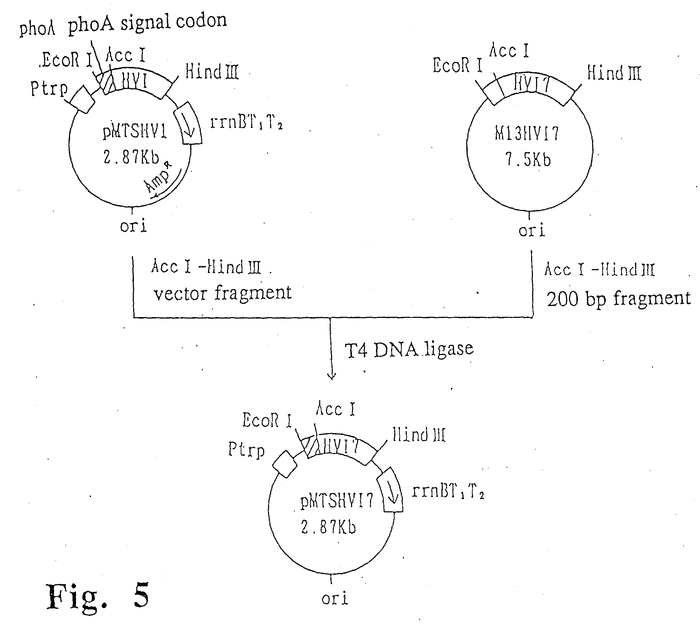

Above mentioned mutated 200 bp DNA fragment and abovementioned fragment of expression vector pMTSHV1 were ligated with T4 DNA ligase (Fig. 5), by which E. coli JM 109 strain was transformed, and mutant expression plasmid pMTSHV17 was obtained. DNA sequence of this plasmid was confirmed by the method of Sanger et al.. Furthermore, E. coli RR1 strain was transformed by using this plasmid, and higher expression than that of JM 109 strain was observed.

### (3) Secretion production of hirudin HV-17 by hirudin mutant HV-17 secreting plasmid

E. coli RR1 transformed with plasmid pMTSHV17 [FERM BP-8268, deposited to Fermentation Research Institute as E. coli RR1/pMTSHV17], purified in abovementioned (2), was cultured in 2 xTY medium (16 g/l bactotryptone, 10 g/l bacto-yeast extract and 5 g/l Nacl, pH 7.2) containing 100 µg/ml of ampicillin. After culturing at 37°C for 24 hours, 1 ml of culture medium was collected. Precipitated cells were suspended in 1ml of 30mM Tris-HCl (pH7.4) containing 25% sucrose and lmM EDTA, followed by treating at room temperature for 10 minutes. After collecting cells by centrifugation, cells were suspended in lml of cold water to release the substance in the periplasm space of cell by osmotic shock.

Then, cells were removed by centrifugation and supernatant was prepared. The amount of accumulated secreted mutant hirudin was determined by measuring its anti-thrombin activity in the supernatant.

The anti-thrombin activity was measured by quantitative colorimetry of degree of inhibition of thrombin's hydrolytic activity on synthetic chromogenic substrate Chromozym TH (Tosylglycil-prolylarginine-4-nitroanilide-acetate, Boehringer-Mannheim).

This reaction was carried out as follows. To the measurement buffer (100mM Tris-HCl (pH8.5), 150mM NaCl and 0.1% polyethyleneglycol-6000), 0.5 units of human-thrombin (product of Sigma) was added, hirudin mutant was added, and pre-incubated at 37°C for 3 minutes. To this substrate, 100 µl of Chromozym TH (product of Boehringer-Mannheim) was added to measure ΔOD405nm/min.

A graph was drawn with amount of added hirudin mutant on horizontal axis and ΔOD405nm/min on vertical axis, and hirudin amount of 100% inhibition of thrombin activity was figured out from the graph, which value was defined as 0.5 anti-thrombin unit (ATU).

As a result, strain utilizing plasmid pMTSHV17 exhibited 1,310,000 ATU of anti-thrombin activity per 1L of the culture medium, which means about 131 mg of HV-17 secretion production to periplasm space was observed.

### (4) Secretion of HV17 into fermentor and culture medium with transformed strain of E. coli RR1/pMTSHV17

By similar method described in abovementioned (3), E. coli RR1 strain transformed with plasmid pMTSHV17 was incubated in 2L of 2xTY medium containing 100 µg/ml ampicillin and 2% glucose in 5L fermentor at 37 °C for 24 hours with aeration and agitation, about 556 mg of HV-17 secretion production per a litter of culture medium was observed.

### (5) Purification of hirudin mutant HV17 from culture medium

After fermentation, 1.6 L of culture medium was collected and centrifuged to separate out cells. After the supernatant was filtered through 3.2 µm filter (product of Pole) to remove cells completely, Hirudin mutant HV-17 was purified by chromatographies in following order.

### a) Anion exchange chromatography

- Column:: QAE-toyopearl column (4.4 x 7cm)
- Running buffer:: 10mM potassium phosphate buffer (pH7.0)
- Elution buffer:: 0.2M Nacl, 10mM potassium phosphate buffer (pH7.0)

### b) Gel filtration chromatography

- Column:: Sephacryl S-100 HR (4.4 x 97 cm)
- Running buffer:: 10mM potassium phosphate buffer (pH7.0)

### c) Anion exchange chromatography

- Column:: DEAE-toyopearl column (4.4 x 40cm)
- Running buffer:: A. 10mM potassium phosphate buffer (pH7.0)
B. 10mM potassium phosphate buffer (pH7.0), 0.3 M NaCl
- Gradient:: B. 0 - 100%/12.5 hours

### d) Reverse phase high performance liquid chromatography

- Equipment:: Waters Delta prep 3000
- Column:: Vydac C4 (4.7 x 30 cm)
- Running buffer:: A. 0.05% trifluoroacetate/water
B. acetonitrile
- Gradient:: B. 10 - 60%/50min.
- Flow rate:: B. 80 ml/min.
-

5 nmol of purified HV-17 was hydrolysed with 6N HCl at 110 °C for 24 hours and analyzed by amino acid analyzer (Beckman 7300). The result is shown in Table 1. Compared with HV-1, HV-17 was confirmed to have two less glutamic acids and two more tyrosines, which was the intention of the mutation.

Anti-thrombin activity was measured according to abovementioned (3), specific activity was 12,000 ATU/mg.

**Table 1**

| Amino Acid | Analyzed value | Theoretical value |
|---|---|---|
| Asx | 8.86 | 9 |
| Thr | 3.86 | 4 |
| Ser | 3.64 | 4 |
| Glx | 11.63 | 11 |
| Gly | 8.82 | 9 |
| Ala | ---- | |
| Cys | 5.63 | 6 |
| Val | 3.33 | 4 |
| Met | ---- | |
| Ile | 1.95 | 2 |
| Leu | 4.11 | 4 |
| Tyr | 4.04 | 4 |
| Phe | 1.00 | 1 |
| His | 1.11 | 1 |
| Lys | 2.73 | 3 |
| Arg | ---- | |
| Pro | 3.28 | 3 |

### Example 10

### Production of sulfated form of hirudin mutant HV-17

Hirudin mutant HV-17, prepared in Example 9 by substituting Glu at 61st and 62nd position to Tyr, was sulfated enzymatically using aryl sulfotransferase under following different conditions.
- Hirudin mutant HV-17:: 0.1 mM
- p-nitrophenylsulfate:: 1.0 mM
- Sulfotransferase:: 10 U/ml
- Magnesium chloride:: 25 mM
- Reaction buffer:: 0.1 M Tris-hydrochloric acid buffer (pH8.6)
- Reaction temperature:: 37 °C
- Reaction time:: 24 hours

Separation and collection of sulfated form was done by HPLC under following condition.
- Column:: Nucleosil 5C₁₈ (4 x 150 mm)
- Running buffer::
- A. 0.1% trifluoroacetate/water
B. acetonitrile
- Gradient:: B. 1 - 60%/60 min.
- Flow rate:: 1.0 ml/min.
- Detection:: 230 nm

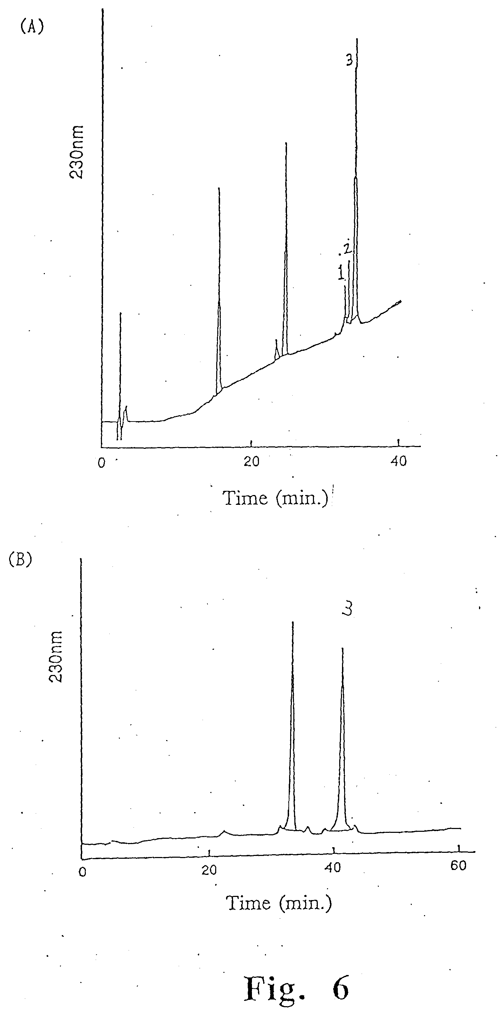

Under this condition, sulfated forms of hirudin mutant HV-17 eluted at 32.4 minutes, 33.0 minutes, 33.7 minutes, respectively, as shown in Fig. 6 (A).

Then, after acetonitorile was removed during the process of concentration, each fraction of sulfated compound was lyophilized.

In case of peak 3, because of its insufficient separation from non-sulfated compound, further separation and collection was done by HPLC under following condition.
- Column:: TSKgel DEAE-5PW (7.5 x 75 mm)
- Running buffer:: A. 20 mM Tris-hydrochloride buffer (pH8.0)
B. 20 mM Tris-hydrochloride buffer (pH8.0) + 0.5 M NaCl
- Gradient:: B. 0 - 100%/50min.
- Flow rate:: 0.8 ml/min.
- Detection:: 230 nm

Under this condition, sulfated forms of hirudin mutant HV-17 eluted at 40.6 minutes as shown in Fig. 6 (B). Then, each fraction of the sulfated compound was concentrated and desalted by reverse phase HPLC, followed by lyophilization.

### Identification of sulfated site

The identification of sulfated site of three kinds of separated and collected sulfated hirudin mutant HV-17 were done by using aminopeptidase M and chymotrypsin.

### a) Amino acid analysis after aminopeptidase M digestion

To 10 µl of 1 mM substrate, 5 µl (250 ng) of α-chymotrypsin (product of Sigma, TLCK treated) was added under icy cold condition, followed by 4 hours digestion in 0.1 M sodium-phoshate buffer (pH7.0) at the temperature of 37°C. To this reaction mixture, 5 µl of aminopeptidase (product of Pierce, 5 mg/ml) was added and additional 18 hours hydrolysis was carried out. Amino acid composition analysis values after the hydrolysis showed peak 1 to be di-sulfated compound, peak 2 and peak 3 to be mono-sulfated compounds.

### b) Peptide mapping by chymotrypsin digestion

To 10 µl of 1 mM substrate, 5 µl (250 ng) of α-chymotrypsin was added under icy cold condition, followed by 24 hours digestion in 0.1 M sodium-phoshate buffer (pH7.0) at the temperature of 37°C. This reaction mixture was loaded on reverse phase HPLC having Nucleosil 5C₁₈ (4 x 150 mm) as stationary phase, and elution points of the digested peptides were confirmed. As the result, when the substrate was non-sulfated hirudin mutant HV-17, two fragments composed of 61 amino acids and four amino acids were generated, by cleavage of amide bond after the 61st Tyr. Similarly, when three kinds of sulfated compounds of peaks 1 - 3 were treated in the same way, in all three cases, the elution point of fragment composed of 61 amino acids were identical to that of non-sulfated peptide. This result shows that in all three kinds of sulfated peptides, 3rd and 61st Tyr were not sulfated. Therefore, putting this result and the result in a) together, peak 1 was confirmed to be disulfated compound having two Tyr residues at 62nd and 63rd sulfated. Also, the elution point of fragment composed of four amino acids, obtained it digestion of peak 2 and peak 3, was identical with the elution point of fragment composed of four amino acids generated by chymotryptic digestion of peak 2 and peak 3 of the case for peptide (B), which fragment is among the fragments generated by chymotrypsin digestion of peaks 1 - 3 in the case of peptide (B). From this result, peak 2 and peak 3 were confirmed to be mono-sulfated compound at 62nd and at 63rd Tyr, respectively.

Above results on identification of sulfation site are summarized below.

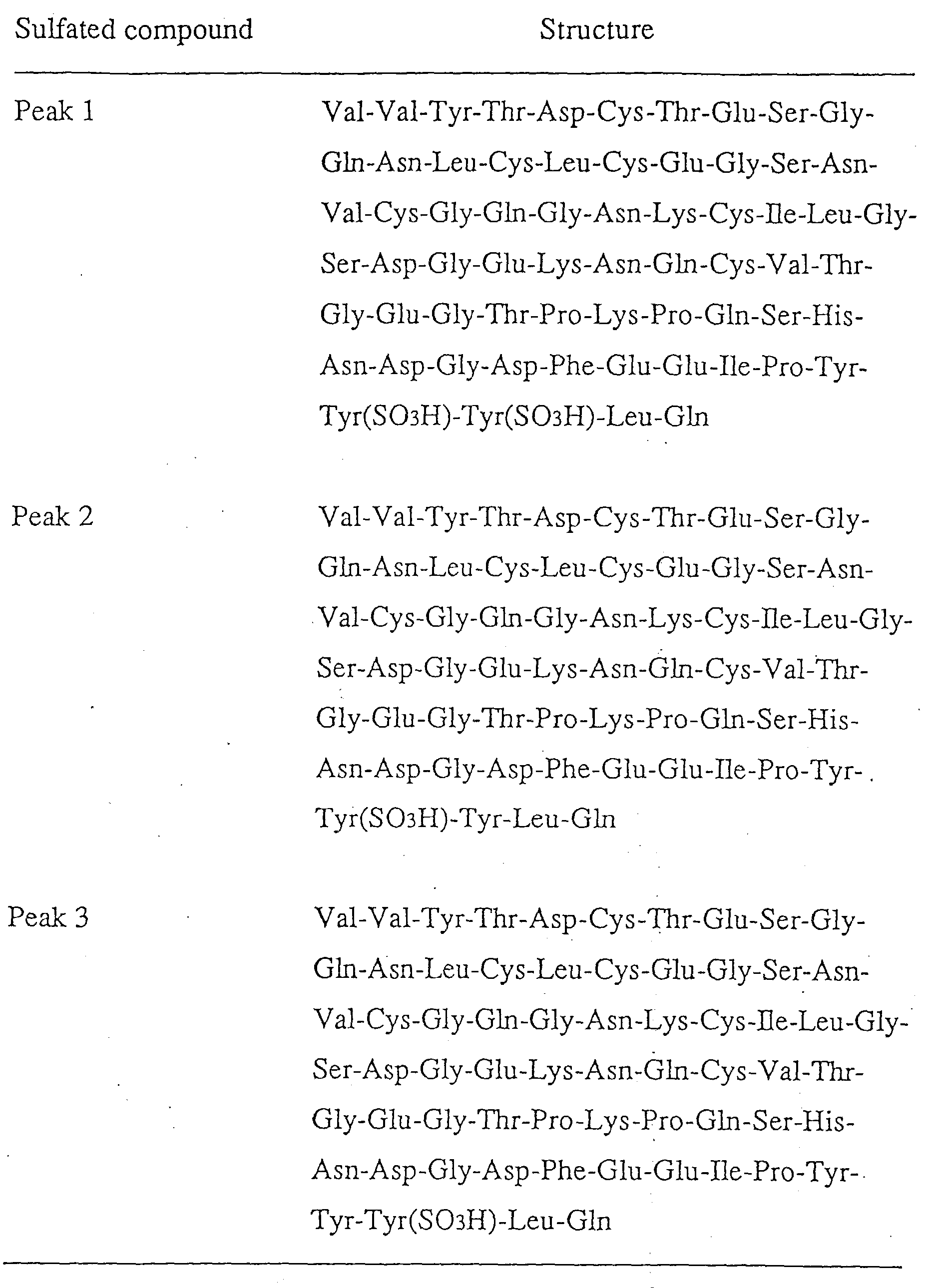

### Example 11

### Example of pharmacological tests (anti-thrombin activity; clotting assay)

Hirudin binds to blood coagulation factor thrombin at 1:1 ratio to inhibit blood coagulation. Recently, binding manner of hirudin and thrombin became clear by crystallography [T. J. Rydel et al., Science, 249, p277-280 (1990)]. The result shows C-terminal region of hirudin binds to thrombin in wrapping manner, and this bond becomes stronger when the hydroxyl group of Tyr at 63rd position is sulfated, which is thought to be the result of increase of anti-thrombin activity.

Some of compounds obtained by the present invention has stronger anti-thrombin activity compared with hirudins produced by conventional method. Measurement of anti-thrombin activity was done by looking at anti-coagulant activity as index using fibrinogen as substrate. That is, dissolved the compounds in 200 µl of 0.1 mM Tris-HCl buffer (pH 7. 5) containing 0.1% polyethyleneglycol-6000, added 100 µl of human-thrombin prepared at 10 NIH units/ml in aforesaid buffer, added 200 µl of human-fibrinogen (product of Sigma) prepared at 6 mg/ml in aforesaid tris-hydrochloride buffer, and coagulation time of the reaction mixtures were measured. For measuring of coagulation times, coagulation time analyzer (Amelung KC10A) was used. Obtained values were converted into units by previously prepared thrombin standard curve. Graphs were prepared by plotting the converted values in vertical axis, and the concentrations of the compounds in horizontal axis. In graphs, thrombin unit was defined as 100 at compound concentration of 0. The concentration of compounds correspond to 50 units were defined as 50% inhibitory concentration of compounds. Results are shown in Table 2.

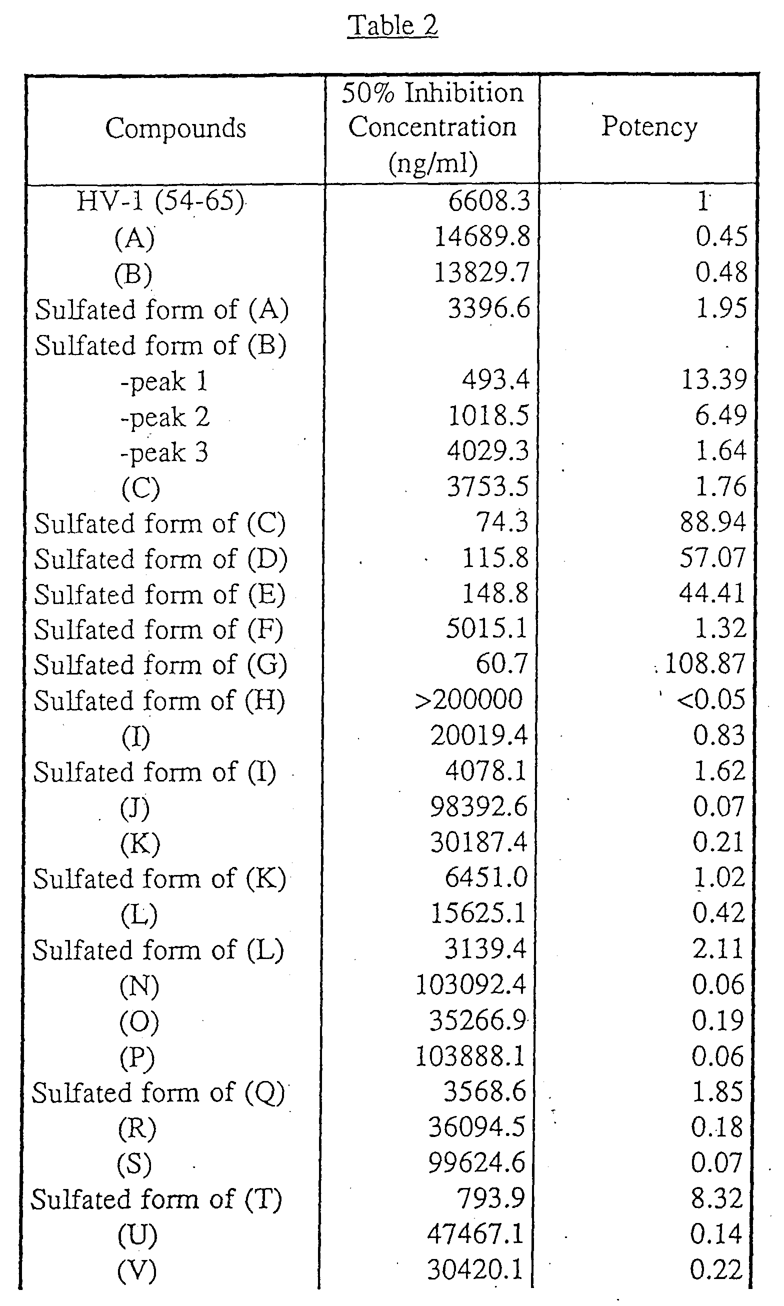

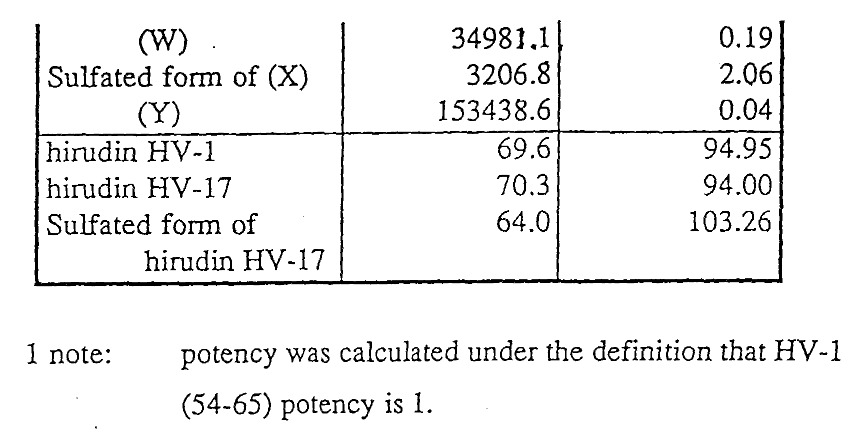

HV-1 (54-65):     H-Gly-Asp-Phe-Glu-Glu-Ile-Pro-Glu-Glu-Tyr-
Leu-Gln-OH

hirudin HV-1:    Val-Val-Tyr-Thr-Asp-Cys-Thr-Glu-Ser-Gly-
Gln-Asn-Leu-Cys-Leu-Cys-Glu-Gly-Ser-Asn-Val-Cys-Gly-
Gln-Gly-Asn-Lys-Cys-Ile-Leu-Gly-Ser-Asp-Gly-Glu-Lys-
Asn-Gln-Cys-Val-Thr-Gly-Glu-Gly-Thr-Pro-Lys-Pro-Gln-
Ser-His-Asn-Asp-Gly-Asp-Phe-Glu-Glu-Ile-Pro-Glu-Glu-
Tyr-Leu-Gln

### Example 12

### Example of pharmacological tests (anti-thrombin activity; chromogenic assay)

Anti-thrombin activities were measured by measuring the degree of inhibition of thrombin's hydrolytic activity on synthetic substrate Chromozym TH (Tosylglycil-prolylarginine-4-nitroanilide-acetate). This reaction was carried out as follows. 350 pM human-thrombin (product of Sigma) was added to the buffer containing 0.1 M Tris-HCl buffer (pH8.5), 150mM NaCl and 0.1% polyethyleneglycol-6000, followed by addition of hirudin HV-1, hirudin HV-17 and separated and collected sulfated hirudin HV-17 (peak 1, peak 2, peak 3), preincubation at 37°C for 3 minutes. Here, the molar concentration of aforementioned human-thrombin was determined by the method of active site titration [G.W. Jameson et al., Biochem. J., 131, p107-l17 (1973)] using 4-methylunberifenyl-p-guanidinobenzoate as substrate. After the preincubation, Chromozym TH (product of Boehringer-Mannheim) was added to a final concentration of 100 µM, and released p-nitroanilide was measured at the wave length of 405 nm, and initial velocity of hydrolytic reactions were measured for each abovementioned hirudins at various concentrations. From this initial velocity of hydrolytic reactions, apparent dissociation constant Ki' were calculated by method of S. R. Stone et al. [Biochemistry, 25, p4622-4628 (1986)]. The results are shown in Table 3.

These results show although hirudin HV-17 is less active than hirudin HV-1, sulfated hirudin HV-17 showed about twice higher activity than hirudin HV-1.

**Table 3**

| Polypeptide | Ki'(pM) | Potency |
|---|---|---|
| hirudin HV-1 | 6.4 | 1 |
| hirudin HV-17 | 16.8 | 0.38 |
| Sulfated form of hirudin HV-17 | | |
| peak 1 | 2.8 | 2.3 |
| peak 2 | 4.3 | 1.5 |
| peak 3 | 5.9 | 1.1 |

### Example 13

### Inhibitory action of sulfated peptides against thrombin induced death

To male mice (20 - 25g), in non-anaesthesia, thrombin (15 NIH units/10g) was administered intravenously, and anti-thrombin activity of tested compound was evaluated by observing disappearance of roll over reflection and death as indexes. All the tested compounds were dissolved in saline and 0.05 ml/10g were administered intravenously 5 min prior to the thrombin injection. The results are shown in Table 4.

**Table 4**

| Tested compound | Amount of administration (mg/kg weight) | Score* |
|---|---|---|
| Thrombin + Saline | | 1.7 |
| (15 NIH units/10g weight) | | |
| | | |
| Thrombin + HV1 | 10 | 1.3 |
| (15 NIH units /10g weight) | 20 | 0.6 |
| | 50 | 0.4 |
| | | |
| Thrombin + sulfated (C) | 20 | 1.2 |
| (15 NIH units/10g weight) | 50 | 0.8 |
| | 100 | 0.6 |

| | | |
|---|---|---|
| *Score score 0: no disappearance of roll over reflection (apparently normal) score 1: disappearance of roll over reflection, but no death within 20 minutes score 2: death within 20 minutes | | |

### Example 14

### Prolongation of bleeding time

Samples were injected into male mice (20 - 25g) from their tail vein under anaesthesia by pentobarbital (40 mg/kg i.p.). A puncture wound was made by inserting a 21G needle (outer diameter 0.85 mm) to the other side of the tail vein after 5 minutes of test compounds administration, and the bleeding time of the wound was measured.

A filter paper was put on the wound with changes in every 15 seconds. Bleeding time is defined as the time required until no red spot is observed on the filter paper. The results are shown in Table 5.

**Table 5**

| Tested compound | Amount of administration (mg/kg weight) | Bleeding time (sec) |
|---|---|---|
| Saline | | 148.5 ± 14.6 |
| | | |
| HV-1 | 2 | 223.5 ± 15.6 |
| | 5 | 376.7 ± 20.2 |
| | 10 | 501.7 ± 47.1 |
| | | |
| Sulfated (C) | 10 | 277.5 ± 31.5 |
| | 20 | 366.0 ± 23,0 |
| | 50 | 432.0 ± 33.3 |

In general, prolongation of bleeding time is one of the side effects of anti-coagulants. Here, the sulfated compound in the present invention was clearly confirmed to have lower bleeding tendency than hirudin HV-1.

### Example 15

### Formulation

(1) Capusles digestible in intestine were prepared using sulfated (C) and (G) obtained in Example 4 with following compositions, respectively.

| | (a) | (b) |
|---|---|---|
| • Main ingredient (sulfated (C) or (G)) | 10 g | 5 g |
| • Lactose | 2.5 g | 7.5 g |
| • Hydroxypropylcellulose | 0.5 g | 0.5 g |

This oral drug may be administered to patients 1 to several times a day.

(2) 5 mg of lyophilized sulfated hirudin mutant HV-17 obtained in Example 10 was dissolved in 10 ml sterilized saline and filled in ampules to produce intravenously injectable drug.

This drug may be administered to patients 1 to several times a day.

### Industrial Applicability

Among the peptides in the present invention, sulfated compounds has extremely high anti-thrombin and anti-platelet activities, which are useful for therapy and prevention of acute deep venous thrombosis, pulmonary thromboembolism, acute arterial embolism of limbs, myocardial infarction etc., that is, they are useful as anti-coagulants. Although non-sulfated compounds themselves have aforesaid anti-thrombin and anti-platelet activities, they are also useful for obtaining peptides of extremely high anti-thrombin and anti-platelet activities by sulfating hydroxyl group of tyrosine residues of said peptides.

### Reference of microorganism

### 1. E. coli RR1/pMTSHV17

Organization of Deposition:
Fermentation Research Institute,
Agency of Industrial Science and Technology,
Ministry of Intemational Trade and Industry

Address:
1-1-3, Tsukuba-shi Higashi, Ibaraki-ken, Japan

Date of Deposition:
February 6, 1991

Deposition Number:
FERM BP-3268

## Claims

1. Hirudin variants or their salts having following amino acid sequence in formula (I) as a part of their sequence or as all of their sequence. Phe-Glu-A-Ile-Pro-B-Tyr(R)-Tyr(R)          (I) [In the formula, A represents Glu or Pro, B represents Glu, Tyr (R), Glu-Asp or Glu-Tyr(R), and (R) represents the hydroxy group or its sulfated ester (-O-SO₃H) of tyrosine residue.]

2. Methods of production of hirudin variants or their salts characterized by sulfating hydroxyl groups of tyrosine residues in amino acid sequence of hirudin variants or their salts having following amino acid sequence in formula (II) as a part of their sequence or as all of their sequence. Phe-Glu-A-Ile-Pro-B-Tyr-Tyr (II) [In the formula, A represents Glu or Pro, B represents Glu, Tyr, Glu-Asp or Glu-Tyr.]

3. Method of production of hirudin variants or their salts according to claim 2, in which sulfation was carried out by aryl sulfotransferase in the presence of sulfate group donors.

4. Method of production of hirudin variants or their salts according to claim 3, in which sulfate group donors are arylsulfates.

5. Method of production of hirudin variants or their salts according to claim 4, in which arylsulfates are one or more than two compounds selected from phenylsulfate, p- or m-nitrophenylsulfate, p- or m- acetylphenylsulfate, tyraminesulfate, p-nitrocatecholsulfate, p-nitrocatecholdisulfate, picosulfate, phenolphthalein disulfate, 4-methylunberiferril sulfate, 1- or 2-naphthyl sulfate, 4-nitro-1-naphthyl sulfate, 4-fenantoryl sulfate, and salts thereof.

6. Method of production of hirudin variants or their salts according to claim 2, in which sulfation was carried out by reaction with sulfurtrioxide complex, or by reaction with sulfuric acid and dicyclohexylcarbodiimide in the presence of sulfate group donors.

7. Anti-coagulants having hirudin variants or their salts described in claim 1 as active ingredients.

## Patentansprüche

1. Hirudin-Varianten oder ihre Salze mit der folgenden Aminosäuresequenz der Formel (I) als Teilsequenz oder als Gesamtsequenz: Phe-Glu-A-Ile-Pro-B-Tyr(R)-Tyr(R) (I) [A bedeutet Glu oder Pro, B bedeutet Glu, Tyr(R), Glu-Asp oder Glu-Tyr(R) und (R) bedeutet eine Hydroxygruppe oder ihren sulfatierten Ester (-O-SO₃H) des Tyrosin-Restes, jeweils in der Formel.]

2. Verfahren zur Herstellung von Hirudin-Varianten oder ihrer Salze, dadurch **gekennzeichnet,** daß man Hydroxylgruppen von Tyrosin-Resten in Aminosäuresequenzen von Hirudin-Varianten oder ihren Salzen sulfatiert, die die folgende Aminosäuresequenz der Formel (II) als Teilsequenz oder als Gesamtsequenz besitzen: Phe-Glu-A-Ile-Pro-B-Tyr-Tyr (II) [A bedeutet Glu oder Pro, B bedeutet Glu, Tyr, Glu-Asp oder Glu-Tyr, jeweils in der Formel.]

3. Verfahren zur Herstellung von Hirudin-Varianten oder ihren Salzen nach Anspruch 2, bei dem man mit Hilfe einer Arylsulfotransferase in Gegenwart eines Sulfatgruppen-Donors sulfatiert.

4. Verfahren zur Herstellung von Hirudin-Varianten oder ihren Salzen nach Anspruch 3, bei dem man als Sulfatgruppen-Donor Arylsulfate verwendet.

5. Verfahren zur Herstellung von Hirudin-Varianten oder ihren Salzen nach Anspruch 4, bei dem man als Arylsulfate eine oder mehr als zwei Verbindungen aus der durch Phenylsulfat, p- oder m-Nitrophenylsulfat, p- oder m-Acetylphenylsulfat, Tyraminsulfat, p-Nitrocatecholsulfat, p-Nitrocatecholdisulfat, Picosulfat, Phenolphthaleindisulfat, 4-Methylunberiferrilsulfat, 1- oder 2-Naphthylsulfat, 4-Nitro-l-naphthylsulfat, 4-Fenantorylsulfat und deren Salze gebildeten Gruppe wählt.

6. Verfahren zur Herstellung von Hirudin-Varianten oder ihren Salzen nach Anspruch 2, bei dem man durch Umsetzen mit einem Schwefeltrioxidkomplex oder durch Umsetzen mit Schwefelsäure und Dicyclohexylcarbodiimid in Gegenwart eines Sulfatgruppen-Donors sulfatiert.

7. Anti-Coagulant mit Hirudin-Varianten oder ihren Salzen gemäß Anspruch 1 als Wirkstoff.

## Revendications

1. Variantes d'hirudine ou leurs sels, ayant la séquence d'acides aminés suivante de formule (I) formant une partie de leur séquence ou la totalité de leur séquence. Phe-Glu-A-Ile-Pro-B-Tyr(R)-Tyr(R)     (I) [Dans la formule, A représente Glu ou Pro, B représente Glu, Tyr(R), Glu-Asp ou Glu-Tyr(R), et (R) représente le groupe hydroxy ou son ester sulfaté (-O-OSO₃H) du résidu tyrosine.]

2. Procédés de production de variantes d'hirudine ou de leurs sels, caractérisés par la sulfatation de groupes hydroxyle de résidus tyrosine dans la séquence d'acides aminés de variantes d'hirudine ou de leurs sels ayant la séquence d'acides aminés suivante de formule (II) formant une partie de leur séquence ou la totalité de leur séquence. Phe-Glu-A-Ile-Pro-B-Tyr-Tyr (II) [Dans la formule, A représente Glu ou Pro, B représente Glu, Tyr, Glu-Asp ou Glu-Tyr.]

3. Procédé de production de variantes d'hirudine ou de leurs sels selon la revendication 2, dans lequel la sulfatation est réalisée par une arylsulfotransférase en présence de donneurs de groupe sulfate.

4. Procédé de production de variantes d'hirudine ou de leurs sels selon la revendication 3, dans lequel les donneurs de groupe sulfate sont des arylsulfates.

5. Procédé de production de variantes d'hirudine ou de leurs sels selon la revendication 4, dans lequel les arylsulfates sont un ou plus de deux composés choisis parmi le phénylsulfate, le p- ou le m-nitrophénylsulfate, le p- ou le m-acétylphénylsulfate, le tyraminesulfate, le p-nitrocatécholsulfate, le p-nitrocatécholdisulfate, le picosulfate, le phénolphtaléninedisulfate, le 4-méthylumbériferrilsulfate, le 1- ou le 2-naphtylsulfate, le 4-nitro-1-naphtylsulfate, le 4-fénantorylsulfate, et leurs sels.

6. Procédé de production de variantes d'hirudine ou de leurs sels selon la revendication 2, dans lequel la sulfatation est réalisée par réaction avec un complexe de trioxyde de soufre, ou par réaction avec de l'acide sulfurique et du dicyclohexylcarbodiimide, en présence de donneurs de groupe sulfate.

7. Anticoagulants renfermant des variantes d'hirudine ou leurs sels selon la revendication 1 comme substances actives.
